# EUROPÄISCHE PATENTSCHRIFT

(11) **EP 3 323 800 B1**
(45) Veröffentlichungstag und Bekanntmachung des Hinweises auf die Patenterteilung: **28.10.2020**
(21) Anmeldenummer: 17201580.2
(22) Anmeldetag: 14.11.2017
(51) Int. Cl.: C07C 41/56, C07C 41/48, C07C 43/11, C07C 41/58

(54) **VERFAHREN ZUR HERSTELLUNG VON POLYOXYMETHYLENDIMETHYLETHERN AUS FORMALDEHYD UND METHANOL IN WÄSSRIGEN LÖSUNGEN**
METHOD FOR PRODUCING POLYOXYMETHYLENE DIMETHYL ETHERS FROM FORMALDEHYDE AND METHANOL IN AQUEOUS SOLUTIONS
PROCÉDÉ DE FABRICATION DES ÉTHERS DIMÉTHYLIQUES DE POLYOXYMÉTHYLÈNE À PARTIR DU FORMALDÉHYDE ET DU MÉTHANOL DANS DES SOLUTIONS AQUEUSES

(30) Priorität: 17.11.2016 DE 102016222657; 28.07.2017 DE 102017213062
(43) Veröffentlichungstag der Anmeldung: 23.05.2018
(73) Patentinhaber: OME Technologies GmbH, 67661 Kaiserslautern (DE)
(72) Erfinder: Burger, Jakob, 94315 Straubing (DE); Schmitz, Niklas, 40235 Düsseldorf (DE); Hasse, Hans, 67661 Kaiserslautern (DE); Ströfer, Eckhard, 68163 Mannheim (DE)
(74) Vertreter: Schuck, Alexander

(56) Entgegenhaltungen:
- WO-A2-00/29365
- DE-A1-102005 027 701
- DE-A1-102005 027 702

## Beschreibung

Die Erfindung betrifft ein Verfahren zur Herstellung von Polyoxymethylendimethylethern (OME) mit ≥ 3 Oxymethylen-Einheiten aus Formaldehyd und Methanol in wässriger Lösung.

OME stellen eine homologe Reihe der allgemeinen Formel CH₃O(CH₂O)ₙCH₃ dar, worin n eine Zahl ≥ 2 bedeutet. Wie das Stamm-Molekül der homologen Reihe, das Methylal CH₃O(CH₂O)ₙCH₃ (n = 1), sind die OME Acetale. Sie werden durch Reaktion von Methanol mit wässrigem Formaldehyd in Gegenwart eines sauren Katalysators hergestellt. Wie andere Acetale sind sie unter neutralen oder alkalischen Bedingungen stabil, werden aber von Säuren angegriffen. Durch Hydrolyse werden sie bei Anwesenheit von Wasser in einem ersten Schritt zu Halbacetalen und Methanol umgesetzt. In einem zweiten Schritt werden die Halbacetale zu Formaldehyd und Methanol umgesetzt. Nachfolgend werden Polyoxymethylendimethylether mit n Oxymethylen-Einheiten als OMEₙ abgekürzt.

Im Labormaßstab werden OME durch Erhitzen von Polyoxymethylenglykol oder Paraformaldehyd mit Methanol in Gegenwart von Spuren von Schwefelsäure oder Salzsäure be
i Temperaturen von 150 bis 180°C und Reaktionszeiten von 12 bis 15 Stunden hergestellt, wie beschrieben in Reuss G., Disteldorf W., Grundler O., Hilt A., Ullmanns Encyclopedia of Industrial Chemistry, Wiley-VCH, Weinheim 1988. Dabei kommt es zu Zersetzungsreaktionen unter Bildung von Kohlendioxid und zur Bildung von Dimethylether. Bei einem Paraformaldehyd oder Polyoxymethylenglykol zu Methanol-Verhältnis von 6:1 werden Polymere mit n > 100, im Allgemeinen n = 300 bis 500 erhalten. Die Produkte werden mit Natriumsulfitlösung gewaschen und anschließend durch Kristallisation fraktioniert. Die Methode der Laborherstellung ist nicht direkt in den technischen Maßstab übertragbar. Die erhaltene Produktmischung liegt außerhalb der Bandbreite anwendungstechnisch interessanter Einsatzgebiete.

OME sind unter anderem von Interesse als Dieselkraftstoff, als Absorptionsmittel zur Abtrennung von Kohlendioxid aus Gasmischungen (Burger J., Ströfer E., Hasse H. Production process for diesel fuel components poly(oxymethylene) dimethyl ethers from methane-based products by hierarchical optimization with varying model depth. Chemical Engineering Research and Design. 2013, 91(12), 2648-2662) oder als sichere Brennstoffe für Brennstoffzellen (Devaux D., Yano H., Uchida H., Dubois J. L., Watanabe M. Electro-oxidation of hydrolysed polyoxymethylene-dimethylether on PtRu supported catalysts Electrochimica Acta.2011, 56(3).,1460-1465). Von besonderem Interesse ist dabei die Verwendung als Dieselkraftstoff, da sowohl OME einer Kettenlänge als auch OME-Mischungen enthaltend OME verschiedener Kettenlängen zur Verringerung der Rußbildung bei der Verbrennung führen. Die OME einer Kettenlänge bzw. die OME-Mischungen können dabei mit konventionellem Dieselkraftstoff gemischt werden oder auch direkt (d.h. ohne Zumischung von konventionellem Dieselkraftstoff) als Kraftstoff eingesetzt werden. Werden andere sauerstoffhaltige Verbindungen, wie z.B. Methanol, zugesetzt, so weisen diese erhebliche Mängel auf. Solche Mängel sind unter anderem die mangelhafte Löslichkeit in Dieselkraftstoff, eine unbefriedigend niedere Cetan-Zahl und ein verminderter Flammpunkt der erhaltenen Zubereitung. Diese Mängel können prinzipiell durch weitere Additive behoben werden. Dies weist jedoch die Nachteile auf, dass die Kosten der Zubereitung um die Kosten der Additive steigen, die Lagerhaltung und Abmischung bei der Herstellung der Zubereitung kompliziert ist, und ferner die Additive die brenntechnischen Eigenschaften der Zubereitung beeinflussen.

Es besteht also ein erheblicher Bedarf, OME ökonomisch und ökologisch günstig in großen Mengen und in hoher Reinheit herzustellen.

US 2,449,469 beschreibt einen Prozess, bei dem Methylal mit Paraformaldehyd oder einer konzentrierten Formaldehyd-Lösung in Gegenwart von Schwefelsäure erhitzt wird. Dabei werden Polyoxymethylendimethylether mit 2 bis 4 Oxymethylen-Einheiten pro Molekül erhalten. Die Beispiele beziehen sich auf diskontinuierliche Prozesse, ein skalierbares technisches Verfahren wird nicht offenbart.

US 5,746,785 beschreibt die Herstellung von Rohmischungen von OME mit einem Molgewicht von 80 bis 350, entsprechend 1 bis 10 Oxymethylen-Einheiten (n = 1 bis 10), durch Reaktion von 1 Teil Methylal mit 5 Teilen CH₂O in Form von Paraformaldehyd in Gegenwart von 0,1 Gew.-% Ameisensäure bei einer Temperatur von 150 bis 240°C, oder durch Reaktion von 1 Teil Methanol mit 3 Teilen CH₂O in Form von Paraformaldehyd bei einer Temperatur von 150 bis 240°C. Die erhaltenen Rohmischungen werden in Mengen von 5 bis 30 Gew.-% einem Dieselkraftstoff zugesetzt. Dabei bilden sich zwei Phasen, wobei die leichtere Phase das fertige, OME enthaltende Produkt ist, welches dem Dieselpool zugeführt wird. Die Verwendung der schwereren Phase wird nicht beschrieben.

US 6,534,685 offenbart die Herstellung von OME mit 2 bis 6 Formaldehyd-Einheiten im Molekül durch Umsetzung von Methylal mit Paraformaldehyd in Gegenwart von Trifluorsulfonsäure. Dabei werden mit einer Selektivität von 94,8 % OME mit n = 2 bis 5 gebildet, wobei zu 49,6 % das Dimer (n = 2) erhalten wird. Die erhaltenen OME werden in Mengen von 4 bis 11 Gew.-% einem Dieselkraftstoff zugesetzt. Es wird lediglich die Synthese der OME beschrieben, ein skalierbares technisches Verfahren wird nicht offenbart.

US 6,392,102 beschreibt die Herstellung von OME durch Umsetzung eines Einsatzstroms enthaltend Methanol und Formaldehyd, welcher durch Oxidation von Dimethylether erhalten wurde, in Gegenwart eines sauren Katalysators und gleichzeitiger Auftrennung der Reaktionsprodukte in einer katalytischen Destillationskolonne. Dabei werden Methylal, Methanol, Wasser und OME erhalten. Das Verfahren weist das Problem auf, dass Wasser im Prozess unter den Verfahrensbedingungen OME hydrolysiert und damit die Prozessausbeute senkt.

Das Wasser wird in den Prozess auf zwei Wegen eingeführt. Der Einsatzstoff Formaldehyd wird in den technisch realisierten und ökonomisch bevorzugten Verfahren zur Formaldehyd-Herstellung durch oxidative Dehydrierung von Methanol (Reaktion 1) hergestellt. Dabei entsteht Wasser als Koppelprodukt.

CH₃OH + (1/2) O₂ → CH₂O + H₂O (Reaktion 1)

Ferner wird auch bei der OME-Synthese aus Formaldehyd und Methanol Wasser gebildet. In solchen Mischungen liegt der Formaldehyd nahezu vollständig chemisch gebunden vor. Formaldehyd reagiert in Anwesenheit von Wasser zu Methylenglykol HO-(CH₂O)-H (Reaktion 2) und Polyoxymethylenglykolen HO-(CH₂O)n-H (Reaktion 3).

CH2O + H2O ↔ HO-(CH2O)-H (Reaktion 2)

HO-(CH₂O)ₙ-1-H + CH₂O ↔ HO-(CH₂O)ₙ-H (Reaktion 3)

Formaldehyd reagiert nach analogem Schema mit Methanol zu den Halbacetalen Hemiformal OH-(CH₂O)-CH₃ (Reaktion 4) und Polyoxymethylenhemiformalen HO-(CH₂O)ₙCH₃ (Reaktion 5).

CH₂O + CH₃OH ↔ OH-(CH₂O)-CH₃ (Reaktion 4)

OH-(CH₂O)ₙ₋₁ - CH₃ + CH₂O ↔ OH-(CH₂O)n-CH₃ (Reaktion 5)

Die instabilen Halbacetale werden auch bei der Hydrolyse von OME gebildet. Diese Halbacetale setzen den Flammpunkt des Dieselkraftstoffgemischs herab und beeinträchtigen somit dessen Qualität. Ein zu niedriger Flammpunkt des Dieselkraftstoffgemischs führt aber dazu, dass die z.B. durch einschlägige DIN-Normen vorgegebenen Spezifikationen nicht mehr erfüllt werden. Halbacetale sind aber wegen vergleichbarer Siedepunkte schwer von den OME abzutrennen. Da die Reaktionen 2 bis 4 reversibel sind und ohne Zugabe eines Katalysators ablaufen, können die Halbacetale potenziell wieder Formaldehyd freisetzen, welcher im Kraftstoff unerwünscht ist.

In dem in US 6,392,102 beschriebenen Verfahren wird die Formaldehydherstellung dahingehend in die OME-Synthese integriert, dass Formaldehyd nicht durch oxidative Dehydrierung von Methanol - wobei im Allgemeinen wässrige Formaldehyd-Lösungen mit einem Formaldehydgehalt von 20 bis 60 Gew.-% erhalten werden - hergestellt wird, sondern durch oxidative Dehydrierung von Dimethylether (Reaktion 6).

CH₃OCH₃ + O₂ → 2 CH₂O + H₂O (Reaktion 6)

Dabei werden Formaldehydgehalte von > 60 Gew.-% erreicht, da Dimethylether formal durch Dehydratisierung zweier Methanol-Moleküle erhalten wird (Reaktion 7) und ein Teil des Wassers bereits bei der Herstellung von Dimethylether abgetrennt werden kann.

2 CH₃OH ↔ CH₃OCH₃ + H₂O (Reaktion 7)

Alternativ kann eine hochkonzentrierte wässrige Formaldehydlösung auch hergestellt werden, indem die wässrige Lösung, wie in US 7,342,139 beschrieben, aufkonzentriert wird und diese konzentrierte Lösung eingesetzt wird. Das prinzipielle Problem des Verfahrens ist damit jedoch nicht gelöst.

Das Verfahren nach US 6,392,102 ist entsprechend technisch komplex und enthält viele Rückführungen. Es umfasst Reaktivdestillationen, mehrere heterogenkatalytische Reaktoren, Destillationskolonnen, Absorptionskolonnen und einen Sprühturm. Dies erfordert hohe Entwicklungs- und Investitionskosten sowie Wartungskosten im laufenden Betrieb.

Die vorstehend beschriebenen Probleme werden vermieden, indem wie in US 7,999,140 und WO 2006/045506 beschrieben, wasserfrei gearbeitet wird. Dies wird durch den Einsatz von Trioxan als wasserfreie formaldehydhaltige Komponente erreicht, welche mit Methylal oder Dimethylether umgesetzt wird. Nun ist der Einsatzstoff Trioxan jedoch teurer als Formaldehyd, da die Herstellung von Trioxan ihrerseits von Formaldehyd als Einsatzstoff ausgeht. Es wird also ein zusätzlicher Verfahrensschritt benötigt.

US 7,671,240 beschreibt ein Verfahren zur Herstellung von OMEₙ₌₃₋₅ durch Umsetzung von Formaldehyd mit Methanol in wässriger Lösung und anschließender destillativer Aufarbeitung des Reaktionsgemisches. Allerdings weist das dort geschilderte Verfahren folgende Nachteile auf: OMEₙ₌₃₋₅ durchlaufen insgesamt drei Destillationskolonnen sowie einen Phasenabscheider, um von den restlichen Komponenten des Reaktoraustrags getrennt zu werden. Aufgrund der Komplexität des reaktiven Stoffgemisches ist es äußerst anspruchsvoll, einen Betriebspunkt zu finden, indem alle Apparate optimal verschaltet sind.

Die Patentschrift enthält keine Beispiele, die einen solchen Betriebspunkt beschreiben.
Insbesondere wird in Schmitz N., Friebel A., von Harbou E., Burger J., Hasse H., Liquid-liquid equilibrium in binary and ternary mixtures containing formaldehyde, water, methanol, methylal, and poly(oxymethylene) dimethyl ethers, Fluid Phase Equilibria. 2016 (425). 127 bis 135 gezeigt, dass sowohl Methanol als auch Formaldehyd, eine starke lösungsvermittelnde Wirkung auf die Mischungslücke zwischen OME und Wasser haben. Es muss daher ein hoher Aufwand betrieben werden, um den Gehalt an Formaldehyd und Methanol vor dem Phasenabscheider destillativ so weit zu senken, dass das System in zwei flüssige Phasen zerfällt. Findet dieser Zerfall nicht statt, ist das Verfahren obsolet.

Nach dem zitierten neuen Stand der Technik wird auch gezeigt, dass die Abtrennung von OME über die organische Phase des Phasenabscheiders nicht quantitativ ist. Ein signifikanter Anteil der OME verlässt den Phasenabscheider über die wässrige Phase. Diese OME müssen weiter aufgearbeitet werden, welches die Komplexität des Gesamtverfahrens erhöht und die Wirtschaftlichkeit senkt.

Dennoch hat die Herstellung von OME aus Methanol und Formaldehyd in wässriger Lösung erhebliche Vorteile gegenüber dem Einsatz anderer Ausgangsstoffe.

Methanol entwickelt sich zu einer auf dem Markt gehandelten und in großen Mengen verfügbaren Plattformchemikalie. Olah (Olah G. A., Goeppert A., Surya Prakash G. K. Beyond oil and gas: the methanol economy, Wiley-VCH, Weinheim 2011) hat ein Szenario entwickelt, wie die Weltenergiewirtschaft auf der Basis von Synthesegas und Methanol neu aufgestellt werden kann. Synthesegas kann sowohl konventionell auf der Basis fossiler Rohstoffe hergestellt werden als auch auf der Grundlage erneuerbarer Energien. Im letzteren Fall wird z. B. Wasser unter Einsatz von solar oder geothermisch erzeugter Elektrizität elektrolytisch gespalten (Reaktion 8).

3 H₂O → 3 H₂ + (3/2) O₂ (Reaktion 8)

Ein Teil des Wasserstoffs wird unter Nutzung von Kohlendioxid in den Synthesebaustein Kohlenmonoxid umgewandelt (Reaktion 9).

CO₂ + H₂ ↔ CO + H₂O (Reaktion 9)

Anschließend werden dann Wasserstoff und Kohlenmonoxid zu den Plattformchemikalien Methanol (Reaktion 10) bzw. Dimethylether (Reaktion 7) umgesetzt.

CO + 2H₂ → CH₃OH (Reaktion 10)

Methanol liegt bei Normalbedingungen in flüssiger Form vor und kann damit einfach gehandhabt und verschifft werden. Methanol kann als Plattformchemikalie in der chemischen Industrie breit eingesetzt werden.

Methanol ist außerdem der bevorzugte Einsatzstoff für die konventionellen Synthesen von Formaldehyd, die in der Fachliteratur (Reuss G., Disteldorf W., Grundler O., Hilt A., Ullmanns Encyclopedia of Industrial Chemistry, Wiley-VCH, Weinheim 1988) geschildert sind. Diese überführen durch oxidative Dehydrierung oder durch einen Zwei-Wege-Mechanismus der oxidativen Dehydrierung gekoppelt mit einer nicht oxidativen Dehydrierung Methanol unter kinetischer Kontrolle in Formaldehyd. Dabei entsteht zwangsläufig immer in gewissen Mengen Wasser (siehe Reaktion 1). Außerdem wird der Reaktoraustrag des Formaldehyd-Reaktors üblicherweise mit einem Überschuss an Wasser in Absorptionskolonnen gequencht. Das Produkt ist eine wässrige Lösung aus Formaldehyd, welche in den meisten Fällen zusätzlich mit Methanol stabilisiert ist.

US 2011/0313202 beschreibt ein Verfahren zur Herstellung von OME aus wässriger Formaldehyd-Lösung und Methanol. In dem Verfahren reagiert zunächst wässriger Formaldehyd teilweise zu Trioxan, wobei eine ionische Flüssigkeit als Katalysator verwendet wird. Die entstehende Mischung enthaltend Formaldehyd, Trioxan und Wasser wird anschließend mit Methanol unter Verwendung einer zweiten ionischen Flüssigkeit als Katalysator zu OME umgesetzt. Der Reaktoraustrag aus dem zweiten Reaktor wird mittels Destillation und eines Phasenabscheiders aufgearbeitet. Nachteilig an dem Verfahren sind erneut die Herstellung des Zwischenprodukts Trioxan und die Verwendung von ionischen Flüssigkeiten als Katalysator. Dies erhöht die Komplexität des Verfahrens. Die Verwendung von ionischen Flüssigkeiten stellt aufgrund ihrer hohen Preise einen wirtschaftlichen Nachteil dar. Sie müssen verlustfrei recycliert werden, was im Allgemeinen sehr aufwendig ist. Des Weiteren ist, wie bereits ausgeführt, die optimale Integration des Phasenabscheiders in den Prozess komplex. Es ist auch unklar, ob Spuren und Abbauprodukte der ionischen Flüssigkeit im Endprodukt verbleiben.

EP 2 987 781 beschreibt ein Verfahren zur Herstellung von OME aus Formaldehyd und Methanol. Die OME werden in einem Zweiphasensystem mit Hilfe eines Extraktionsmittels aus dem Reaktionssystem entfernt. Bevorzugt wird, wie schon in US 5,746,785, Dieselkraftstoff als Extraktionsmittel eingesetzt. Die Wasserentfernung erfolgt aus der wässrigen Phase des Zweiphasensystems mittels z.B. Adsorption an Molekularsieben, Trocknung mit wasserfreien Salzen oder nach einem Membranverfahren. Nachteilig an dem Verfahren ist die Verwendung eines zusätzlichen prozessfremden Extraktionsmittels, welches möglicherweise in Spuren im OME verbleibt. Außerdem werden OME, wie in den Abbildungen 3 bis 6 der Schrift gezeigt, nicht quantitativ aus dem Reaktionssystem entfernt. Dadurch erhöhen sich die Rückführströme des Prozesses und die Wirtschaftlichkeit des Prozesses wird beeinträchtigt.

US 2014/0114093 beschreibt ein Verfahren zur Herstellung von Polyoxymethylendialkylethern, insbesondere OME, in dem durch Reaktion von Formaldehyd und Methanol, unter Verwendung einer ionischen Flüssigkeit als Katalysator, OME gebildet werden. Das Reaktionsgemisch wird anschließend extraktiv und destillativ aufgearbeitet. Die Wasserentfernung erfolgt durch Verdampfung des Wassers aus der schweren katalysatorreichen Phase nach der Extraktion. Nachteilig an dem Verfahren sind wiederum die Verwendung einer ionischen Flüssigkeit als Katalysator sowie die Verwendung von prozessfremden Extraktionsmitteln.

US 2015/0094497 beschreibt ein Verfahren zur Herstellung von OME aus wässriger Formaldehyd Lösung und Methanol. Dabei wird wässrige FormaldehydLösung zunächst konzentriert und mittels Vakuumtrocknung zu festem Paraformaldehyd getrocknet. Paraformaldehyd reagiert anschließend mit Methanol unter Verwendung einer ionischen Flüssigkeit als Katalysator zu OME. Der Reaktoraustrag wird destillativ und extraktiv aufgearbeitet. Nachteilig an dem Verfahren sind die Handhabung des Feststoffes Paraformaldehyd, die Verwendung eines prozessfremden Extraktionsmittels, die Verwendung von ionischen Flüssigkeiten als Katalysator und die Komplexität des Gesamtverfahrens bestehend aus Aufkonzentrierung und Trocknung der wässrigen FormaldehydLösung, chemischer Reaktion, Extraktion und Destillation. Prinzipiell gilt unter Fachleuten, dass Feststoffe in Prozessen zu vermeiden sind, da die Handhabung schwierig und teuer ist.

US 6,534,685 beschreibt eine flüssige Zubereitung aus OMEₙ₌₂₋₅ und einem Dieselkraftstoffgemisch mit dem Siedebereich 150°C bis 350°C. Nachteilig an dieser Zubereitung ist die Verwendung von OME nur einer Kettenlänge oder von synthetischen Mischungen von OME verschiedener Kettenlängen. Dies erfordert zunächst eine aufwendige destillative Trennung der OME nach ihrer Kettenlänge, ohne dass sich dabei ein zusätzlicher Nutzen für das Kraftstoffgemisch enthaltend OME ergibt.

In der wissenschaftlichen Literatur gibt es neuere Publikationen zum Thema OME, von denen sich die meisten mit der Optimierung des Reaktionssystems beschäftigen. Schmitz et al. (Schmitz N., Homberg F., Berje J., Burger J., Hasse H. Chemical equilibrium of the synthesis of poly(oxymethylene) dimethyl ethers from formaldehyde and methanol in aqueous solutions. Industrial and Engineering Chemistry Research. 2015, 54 (25). 6409-6417.; Schmitz N., Burger J., Ströfer, E. , Hasse, H. From methanol to the oxygenated diesel fuel poly(oxymethylene) dimethyl ether: An assessemnt of the production costs. Fuel. 2016 (185), 67 - 72; geben einen Überblick über die Literatur und die Wirtschaftlichkeit. Schmitz et al. (Schmitz N., Homberg F., Berje J., Burger J., Hasse H. Chemical equilibrium of the synthesis of poly(oxymethylene) dimethyl ethers from formaldehyde and methanol in aqueous solutions. Industrial and Engineering Chemistry Research. 2015, 54 (25). 6409-6417.) untersuchen auch das chemische Gleichgewicht der OME-Synthese aus wässrigem Formaldehyd und Methanol. Es wird beschrieben, dass die Kettenlängenverteilung der OME im Reaktoraustrag durch das chemische Gleichgewicht bestimmt ist.

Der OME-Reaktor wird dabei so dimensioniert, dass das chemische Gleichgewicht weitgehend erreicht wird. Weitgehend bedeutet hier, dass die Abweichung der Konzentration jedes einzelnen OME-Oligomers von seiner jeweiligen Gleichgewichtskonzentration < 30%, bevorzugt < 10%, ganz besonders bevorzugt < 5% beträgt. Es ist aber ebenfalls möglich, den Reaktor so zu dimensionieren, dass das chemische Gleichgewicht weitgehend nicht erreicht wird und die Oligomerenverteilung der OME kinetisch kontrolliert ist (Schmitz N.,, Burger J., Hasse H. Reaction kinetics of the formation of of poly(oxymethylene) dimethyl ethers from formaldehyde and methanol in aqueous solutions. Industrial and Engineering Chemistry Research. 2015, 54 (50). 12553-12560.). Die Oligomerenverteilung im Produkt OME ist maßgeblich durch die Führung der Reaktion im Reaktor bestimmt. Eine wirtschaftlich optimale Herstellung von OMEₙ₌₃₋₆ als Kraftstoffzusatz ist dadurch gekennzeichnet, dass die Kettenlängenverteilung der Oligomere im Bereich n = 3 bis 6 im Kraftstoff innerhalb von < 15% Abweichung, bevorzugt innerhalb < 10%, ganz besonders bevorzugt < 5% Abweichung, ihrer Kettenlängenverteilung im Reaktoraustrag entspricht. Nachfolgende Trenn- und Aufarbeitungsschritte schneiden aus der OME-Fraktion des Reaktoraustrags bevorzugt nur OME-Oligomere zu geringer (n < 3) oder zu großer Kettenlänge (n > 6) ab und führen sie zurück. Diese Oligomere zu geringer oder zu großer Kettenlänge haben aufgrund ihrer physikalischen Eigenschaften einen negativen Einfluss auf Spezifikationsmerkmale von OME und der daraus hergestellten Zubereitungen. Solche negativen Einflüsse sind z. B. ein zu niedriger Flammpunkt bei geringen Kettenlängen oder ein zu hoher Cold Filter Plugging Point bei zu großen Kettenlängen in der Abmischung mit Dieselfraktionen. Sollen aufgrund von Spezifikationsfestsetzungen oder Normungen Oligomere mit n < 3 und/oder n > 6 im Produkt verbleiben, so kann deren Anteil über die Schnittschärfe der Trenn- und Aufarbeitungsschritte und Recyclisierungen oder durch Zumischung eingestellt werden.

Damit ist OME ein "product by process", da der Reaktor die Spezifikation hinsichtlich der Oligomerenverteilung vorbestimmt. Durch die folgenden Trenn- und Aufarbeitungsschritte wird die Spezifikation endgültig festgelegt, da zu hohe und zu niedere Oligomere von OME abgeschnitten und im Prozess recycliert werden.

Analog haben Burger et al. (Burger J., Ströfer E., Hasse H., Production process for diesel fuel components poly(oxymethylene) dimethyl ethers from methane-based products by hierarchical optimization with varying model depth. Chemical Engineering Research and Design, 2013, 91(12), 2648-2662) für einen thermodynamisch bestimmten idealisierten Gesamtprozess für die OME-Synthese aus Trioxan und Methylal eine natürliche Oligomerenverteilung des OME gefunden von OME₃: 0,43 g/g, OME₄: 0,34 g/g, OME₅: 0,22 g/g OME₆: < 0,01 g/g. Es besteht also nach wie vor ein Bedarf an Verfahren zur Herstellung von OME, welche von den handelsüblichen, preiswerten und in großen Mengen leicht verfügbaren Standardprodukten Formaldehyd und Methanol in wässriger Lösung ausgehen. Vor dem Hintergrund ihrer Bedeutung als Dieselkraftstoffkomponenten besteht insbesondere ein Bedarf an der selektiven und wirtschaftlichen Herstellung von OME mit 3 bis 6 Oxymethylen-Einheiten (OMEₙ₌₃₋₆).

Aufgabe der Erfindung ist es daher, ein verbessertes Verfahren zur Herstellung von OMEₙ₌₃₋₆ bereitzustellen, welches von den günstig verfügbaren Edukten Formaldehyd und Methanol ausgeht.
Gelöst wird die Aufgabe durch ein Verfahren zur Herstellung von Polyoxymethylendimethylethern mit ≥ 3 Oxymethylen-Einheiten (OME_{n≥3}) mit den Schritten (i) und (ii):
(i) Einspeisung von Formaldehyd, Methanol und Wasser in einen Reaktor R und Umsetzung zu einem Reaktionsgemisch enthaltend Formaldehyd, Wasser, Methylenglykol (MG), Polyoxymethylenglykole (MGₙ), Methanol, Hemiformale (HF), Methylal (OMEₙ₌₁) und Polyoxymethylendimethylether (OME_{n>1});
(ii) Einspeisung des Reaktionsgemisches in eine Reaktivdestillationskolonne K1 und Auftrennung in eine Leichtsiederfraktion F1 enthaltend Formaldehyd, Wasser, Methylenglykol (MG), Polyoxymethylenglykole (MGₙ), Methanol, Hemiformale (HF), Methylal (OMEₙ₌₁) und Polyoxymethylendimethylether mit 2 bis 3 Oxymethylen-Einheiten (OMEₙ₌₂₋₃) und eine Schwersiederfraktion F2, enthaltend im Wesentlichen Polyoxymethylendimethylether mit mehr als zwei Oxymethylen-Einheiten (OMEn≥3), wobei die Schwersiederfraktion F2 mindestens 90 Gew.-% Polyoxymethylen-dimethylether mit mehr als zwei Oxymethylen-Einheiten (OMEn≥3) enthält.

Bevorzugt ist ein Verfahren zur Herstellung von Polyoxymethylendimethylethern mit 3 bis 6 Oxymethylen-Einheiten (OMEₙ₌₃₋₆) mit den zusätzlichen Schritten (iii) und (iv):
(iii) Einspeisung der Schwersiederfraktion F2 in eine Destillationskolonne K2 und Auftrennung in eine Produktfraktion F3 enthaltend Polyoxymethylendimethylether mit 3 bis 6 Oxymethylen-Einheiten (OMEₙ₌₃₋₆) und eine Schwersiederfraktion F4 enthaltend Polyoxymethylendimethylether mit mehr als 6 Oxymethylen-Einheiten (OME_{n>6});
(iv) gegebenenfalls Rückführung der Schwersiederfraktion F4 in den Reaktor R.

Vorzugsweise umfassen die vorstehend definierten Verfahren auch noch den Schritt (v), insbesondere die Schritte (v) und (vi):
(v) Einspeisung der Leichtsiederfraktion F1 in einen Apparat zur Wasserabtrennung und Abtrennung von Wasser oder einer wasserreichen Fraktion F6, wobei ein wasserarmer Strom F5 erhalten wird;
(vi) gegebenenfalls Rückführung des wasserarmen Stroms F5 in den Reaktor.

Das erfindungsgemäße Verfahren, das nach intensiver Forschung gefunden wurde, löst überraschenderweise die oben aufgezeigten, bei einem OME-Herstellverfahren ausgehend von den Edukten Methanol und Formaldehyd in wässriger Lösung auftretenden grundlegenden Probleme. Nach dem Reaktor werden sowohl neue als auch konventionelle Trennschritte eingeführt und auf geschickte Art und Weise so miteinander kombiniert, dass ein ökonomisch und ökologisch vorteilhafter Prozess resultiert.

Vorteilhafte Ausgestaltungen des Verfahrens sind dadurch gekennzeichnet, dass
- Katalysatoren nur in fester Phase vorliegen und damit einfach von dem fluiden Reaktionsmedium getrennt werden können;
- alle anderen Stoffe außer den Katalysatoren stets nur in fluiden Phasen als Gas bzw. Flüssigkeit vorliegen;
- auf die Einführung von zusätzlichen Hilfsstoffen zur Katalyse, Extraktion oder Stofftrennung verzichtet wird;
- nach dem OME-Reaktor eine spezielle Reaktivdestillationskolonne K1 eingeführt ist, in der die OME der Kettenlänge n ≥ 3 vom Reaktoraustrag abgetrennt werden, wobei eine Leichtsiederfraktion verbleibt;
- die Reaktivdestillationskolonne K1 üblicher Bauart ist. Als trennwirksame Einbauten werden verwendet entweder Böden, Packungen oder Füllkörper;
- der Druck in der Reaktivdestillationskolonne K1 zwischen 0,2 bar und 5 bar, bevorzugt zwischen 0,5 bar und 4 bar, besonders bevorzugt zwischen 1 bar und 3 bar, beträgt;
- die Kopftemperatur in der Reaktivdestillationskolonne K1 zwischen 25°C und 125°C beträgt, bevorzugt zwischen 45°C und 115°C beträgt, besonders bevorzugt zwischen 65°C und 105°C beträgt;
- die Sumpftemperatur in der Reaktivdestillationskolonne K1 zwischen 110°C und 255°C beträgt, bevorzugt zwischen 140°C und 240°C beträgt, besonders bevorzugt zwischen 170°C und 225°C beträgt;
- der Durchmesser der Reaktivdestillationskolonne K1 durch den Durchsatz der Kolonne bestimmt wird. Die Berechnung erfolgt nach den dem Fachmann bekannten Methoden. Der Durchsatz der Kolonne skaliert mit der Kapazität der OME Anlage;
- die Höhe der Kolonne zwischen 10 und 40 theoretischen Trennstufen, bevorzugt zwischen 15 und 30 theoretischen Trennstufen, besonders bevorzugt zwischen 20 und 25 theoretischen Trennstufen ist. Die Umrechnung auf die Kolonnenhöhe in Metern erfolgt für jede der beschriebenen trennwirksamen Einbauten (Böden, Packungen, Füllkörper) nach den dem Fachmann bekannten Methoden.
- unterhalb des Zulaufs die Höhe der Reaktivdestillationskolonne K1 zwischen 3 und 20 theoretischen Trennstufen, bevorzugt zwischen 4 und 15 theoretischen Trennstufen, besonders bevorzugt zwischen 5 und 10 theoretischen Trennstufen beträgt. Die Umrechnung auf die Höhe des Zulaufs in Metern erfolgt für jede der beschriebenen trennwirksamen Einbauten (Böden, Packungen, Füllkörper) nach den dem Fachmann bekannten Methoden;
- das Rücklaufverhältnis in der Reaktivdestillationskolonne K1 mindestens 0,20 g/g, beispielsweise mindestens 0,30 g/g oder mindestens 0,40 g/g beträgt;
- das Verhältnis aus Volumen der Flüssigkeit im Bereich zwischen Zulauf und Sumpfabzug inklusive möglicher Seitenreaktoren und des Sumpfes zum Volumenstrom des Sumpfabzugs in der Reaktivdestillationskolonne K1 mindestens 10 min beträgt; dieses Verhältnis kann auch mehr als 15 min oder mehr als 30 min betragen;
- die Reaktivdestillationskolonne und deren Einbauten aus üblichen Materialien bestehen, bevorzugt ist Stahl;
- der pH-Wert in der Reaktivdestillationskolonne K1 im Bereich zwischen Zulauf und Sumpfabzug, inklusive möglicher Seitenreaktoren und des Sumpfes, zwischen 4 und 14, bevorzugt zwischen 5 und 14, besonders bevorzugt zwischen 7 und 14 ist;
- der pH-Wert in der Reaktivdestillationskolonne K1 im Bereich zwischen Zulauf und Kopfabzug keine wesentliche Rolle spielt;
- die Regelung des pH-Wertes in der Reaktivdestillationskolonne K1, falls der pH-Wert zu niedrig ist, im Bereich zwischen Zulauf und Sumpfabzug durch eine Laugedosierung erfolgt, bevorzugt durch eine Dosierung von Natriummethanolat in methanolischer Lösung;
- der Zulauf zu den Kolonnen sowohl flüssig als auch gasförmig oder beides sein kann. Nassdampf ist daher ebenfalls möglich;
- nach der Reaktivdestillationskolonne K1 eine nachgeschaltete Destillationskolonne K2 üblicher Bauart installiert ist, die OME der Kettenlänge n ≥ 3 in das Produkt OMEₙ₌₃₋₆ und eine schwersiedende Fraktion OME_{n>6} trennt;
- die schwersiedende Fraktion der Kolonne K2 in den OME-Reaktor zurückgeführt wird;
- die Wasserabtrennung aus der Leichtsiederfraktion in Kopplung mit einer nachgeschalteten Trenneinheit geschieht;
- die Trenneinheit zur Abtrennung des Wassers eine dem Fachmann bekannte Einrichtung, bevorzugt eine Pervaporations-, Dampfpermeations- oder Adsorptionsanlage, ist;
- der wasserarme Strom aus der Wasserabtrennung in den Reaktor zurückgeführt wird;
- optional der wasserreiche Strom aus der Wasserabtrennung enthaltend neben Wasser auch Formaldehyd, Methanol, Methylal, und wenig OME_{n = 2-3} weiter aufgearbeitet wird;
- die optionale Aufarbeitung des wasserreichen Stroms aus der Wasserabtrennung derart gestaltet ist, dass Methanol und Methylal als Kopfprodukt einer Destillationskolonne K3 gewonnen werden und in den OME Reaktor zurückgeführt werden und das Sumpfprodukt enthaltend Wasser, Formaldehyd und wenig OMEₙ₌₂₋₃ vorzugsweise in eine Formaldehyd-Anlage zurückgeführt wird;
- die wässrige Lösung aus Formaldehyd und Methanol als Zulaufstrom dem Reaktor zugeführt wird,
- Abgasströme aus den verschiedenen Verfahrensschritten in eine Formaldehyd-Anlage zurückgeführt werden können,
- das Verfahren "auf der grünen Wiese" außerhalb eines konventionellen Standortes errichtet und betrieben werden kann, aber vorzugsweise im direkten Verbund mit einer Formaldehyd-Anlage betrieben wird. Dabei ist es nicht notwendig, dass die Formaldehyd-Anlage die gesamte benötigte Menge an Formaldehyd zur OME-Synthese zur Verfügung stellt, sie sollte aber in der Lage sein, die verdünnten Rückführströme aus der OME-Anlage zur stofflichen Verwertung aufzunehmen;
- das Verfahren im Verbund mit anderen Verfahren an einem Verbundstandort errichtet und betrieben werden kann, bevorzugt im Verbund mit einer Methanol- und Formaldehyd-Anlage und/oder einer Raffinerie, bevorzugt im Verbund mit einer Formaldehyd-Anlage, ganz besonders bevorzugt im Verbund mit einer Formaldehyd-Anlage, die nach dem Silberkontaktverfahren arbeitet. Alternativ können die Edukte ganz oder anteilig über eine Pipeline, Schiffe oder andere Logistikwege bereitgestellt werden und die Produkte entsprechend verschifft werden;
- das hergestellte Produkt in z.B. den oben erwähnten Anwendungen eingesetzt werden kann, wobei es insbesondere alleine oder anteilig als Dieselkraftstoff Verwendung finden kann und wobei die anteilige Einbringung in andere Dieselkraftstoffe oder deren Mischungen durch statisches oder dynamisches Mischen in dem Fachmann bekannten Einrichtungen erfolgt.

In einer bevorzugten Ausführungsform weist die Kolonne K1 im Betrieb folgende Merkmale auf
- der Druck in der Reaktivdestillationskolonne K1 beträgt von 0,2 bar bis 5 bar;
- die Kopftemperatur in der Reaktivdestillationskolonne K1 beträgt von 25°C bis 125°C;
- die Sumpftemperatur in der Reaktivdestillationskolonne K1 beträgt von 110°C bis 255 C;
- die Reaktivdestillationskolonne K1 weist 10 bis 40 theoretische Trennstufen, bevorzugt 15 bis 30 theoretische Trennstufen, besonders bevorzugt 20 bis 25 theoretische Trennstufen auf;
- unterhalb des Zulaufs weist die Reaktivdestillationskolonne K1 3 bis 20 theoretische Trennstufen auf;
- das Rücklaufverhältnis in der Reaktivdestillationskolonne K1 beträgt mindestens 0,20 g/g;
- das Verhältnis aus Volumen der Flüssigkeit im Bereich zwischen Zulauf und Sumpfabzug einschließlich der gegebenenfalls vorhandenen Seitenreaktoren und des Sumpfes zum Volumenstrom des Sumpfabzugs in der Reaktivdestillationskolonne K1 beträgt mindestens 10 min;
- der pH-Wert in der Reaktivdestillationskolonne K1 im Bereich zwischen Zulauf und Sumpfabzug einschließlich der gegebenenfalls vorhandenen Seitenreaktoren und des Sumpfes ist zwischen 4 und 14 .

In einer besonders bevorzugten Ausführungsform weist die Kolonne K1 im Betrieb folgende Merkmale auf:
- der Druck in der Reaktivdestillationskolonne K1 beträgt von 0,5 bis 4 bar;
- die Kopftemperatur in der Reaktivdestillationskolonne K1 beträgt von 45 bis 115°C;
- die Sumpftemperatur in der Reaktivdestillationskolonne K1 beträgt von 140 bis 240°C;
- die Reaktionsdestillationskolonne K1 weist 5 bis 30 theoretische Trennstufen auf;
- unterhalb des Zulaufs weist die Reaktivdestillationskolonne K1 4 bis 15 theoretische Trennstufen auf;
- der pH-Wert in der Reaktivdestillationskolonne K1 im Bereich zwischen Zulauf und Sumpfabzug ist zwischen 5 und 14.

In einer insbesondere bevorzugten Ausführungsform weist die Kolonne K1 im Betrieb folgende Merkmale auf:
- der Druck in der Reaktivdestillationskolonne K1 beträgt von 1 bis 3 bar;
- die Kopftemperatur in der Reaktivdestillationskolonne K1 beträgt von 65 bis 105°C;
- die Sumpftemperatur in der Reaktivdestillationskolonne K1 beträgt von 170 bis 225°C;
- die Reaktionsdestillationskolonne K1 weist 20 bis 25 theoretische Trennstufen auf;
- unterhalb des Zulaufs weist die Reaktivdestillationskolonne K1 5 bis 10 theoretische Trennstufen auf;
- der pH-Wert in der Reaktivdestillationskolonne K1 im Bereich zwischen Zulauf und Sumpfabzug ist zwischen 7 und 14.

Katalysatoren in fester Phase in Kombination mit flüssigen oder gasförmigen Einsatzstoffen haben den Vorteil der leichten Abtrennbarkeit des Katalysators von der flüssigen oder gasförmigen Phase. Katalysatoren in fluider Phase können oft nicht vollständig von der fluiden Phase der Reaktionsmischung abgetrennt werden. Damit verbleiben sie zum Teil in den Reaktionsprodukten und können zu einer Minderung der Produktqualität führen. Ferner können sie in Apparaten zu Niederschlägen, Ablagerungen und Fouling führen. Fouling mindert die Verfügbarkeit und damit die Kapazität der Anlage. Durch die prinzipiell unvollständige Trennung des Katalysators von der Reaktionsmischung, muss dieser stets nachführend ergänzt werden. Die Recyclierungsrate eines Katalysators in fluider Phase ist innerhalb eines Verfahrens immer < 100%. Dies senkt aufgrund der Verluste des teuren Katalysators nachhaltig die Wirtschaftlichkeit des Gesamtverfahrens. Dies gilt für Verfahren, die zum Beispiel flüssige ionische Flüssigkeiten als Katalysator nutzen oder ein separates Lösungsmittel einführen, in dem ein spezieller homogener Katalysator gelöst ist.

Generell gilt, dass zur Optimierung der Wirtschaftlichkeit und zur Optimierung und Sicherstellung der hohen Produktqualität auf Hilfsstoffe jeder Form so weit wie möglich verzichtet werden sollte.

Dem Fachmann ist bekannt, dass die Handhabung von flüssigen und gasförmigen Stoffen in verfahrenstechnischen Apparaten bevorzugt ist. Sie lassen sich einfach fördern. Die entsprechenden Apparate sind in verschiedensten Ausführungen in großer Zahl erhältlich. Die Apparate sind keine Sonderkonstruktionen und sind weitgehend risikolos skalierbar. Entsprechend können Planung und Errichtung einer Großanlage wirtschaftlich günstig durchgeführt werden. Die Skalierung des Gesamtverfahrens ist damit durchgängig möglich. Dies hebt wirtschaftliche Vorteile entsprechend der dem Fachmann geläufigen Regeln der "economy of scale". Destillationskolonnen sind in der Auslegung technischer Verfahren als Apparate generell bevorzugt, da Destillationskolonnen die häufigste Art der Stofftrennung in der chemischen Technik sind. Die Methoden zur Auslegung sind im Allgemeinen. weitgehend ausgereift.

Dem Fachmann ist bekannt, dass bei der Destillation von formaldehydhaltigen Mehrkomponentengemischen enthaltend Wasser und Methanol Reaktionen zwischen Wasser, Methanol und Formaldehyd zu Polyoxymethylenglykolen und Polyoxymethylenhemiformalen führen (siehe Reaktionen 2-5). Die entsprechenden Reaktionen sind in der Literatur beschrieben (Hahnenstein I., Albert M., Hasse H., Kreiter C. G., Maurer G., NMR Spectroscopic and Densimetric Study of Reaction Kinetics of Formaldehyde Polymer Formation in Water, Deuterium Oxide, and Methanol, Industrial and Engineering Chemistry Research 1995, 34(2), 440-450). Diese Mischungen weisen ein komplexes Phasenverhalten auf. Es bilden sich zahlreiche (reaktive) Azeotrope, welche die Auftrennung des Reaktionsgemisches erschweren.

Es wurde nun überraschenderweise gefunden, dass sich OME mit 3 und mehr Oxymethylen-Einheiten (OME_{n≥3}) in einem einzigen destillativen Trennschritt von den übrigen Komponenten des Reaktoraustrags quantitativ abtrennen lassen. Quantitativ bedeutet hier, dass mindestens 80%, bevorzugt mindestens 85%, besonders bevorzugt mindestens 90% der OME_{n≥3} vom Reaktoraustrag getrennt werden. Dies ist aufgrund der ähnlichen Siedepunkte der OME_{n≥3} und der im Reaktoraustrag zwangsläufig enthaltenden Polyoxymethylenglykole (n ≥ 3) und Polyoxymethylenhemiformale (n≥ 3) zunächst nicht zu erwarten. Durch das komplexe Zusammenspiel von Reaktionen und Destillation reagieren Polyoxymethylenglykole (n ≥ 3) und Polyoxymethylenhemiformale (n≥ 3) in der Kolonne offensichtlich zu den analogen Spezies kürzerer Kettenlänge, die sich nun sehr wohl von den OME_{n≥3} destillativ trennen lassen.

Bei der Kolonne K2 handelt es sich im Allgemeinen um eine konventionelle Destillationskolonne zur physikalischen Trennung von Stoffgemischen, da der Zulaufstrom zur Kolonne K2 im Wesentlichen kein Formaldehyd, Methanol und Wasser mehr enthält.

Die Folgeschritte zur Wasserabtrennung durch insbesondere Pervaporation, Dampfpermeation und/oder Adsorption sind verfahrenstechnisch etablierte Grundoperationen und werden z.B. zur Entwässerung der Kraftstoffkomponente Bioethanol genutzt, wie beschrieben in Frolkova A. K., Raeva V. M. Bioethanol dehydration: state of the art, Theoretical Foundations of Chemical Engineering, 2010 44 (4), 545-556.

Insbesondere beschreibt US 2015/0053616 die Möglichkeit der Entwässerung von wässrigen Lösungen enthaltend Formaldehyd und OME mittels Pervaporation. Die Möglichkeit der Entwässerung von Lösungen enthaltend Formaldehyd, Methanol und Wasser mittels Adsorption ist ebenfalls bekannt, wie beschrieben in Hasse H., Dampf-Flüssigkeitsgleichgewichte, Enthalpien und Reaktionskinetik in formaldehydhaltigen Mischungen, Ph. D Thesis, Universität Kaiserslautern, Kaiserslautern 1990.

Die Skalierbarkeit von Adsorptions- und Membranverfahren in den großtechnischen Maßstab ist gegeben. So sind Adsorptionsanlagen mit etwa 200 t Adsorptionsmittel technisch realisiert, wie beschrieben in Baerns M., Behr, A., Brehm A., Gmehling J., Hoffmann H., Onken U., Renken A., Technische Chemie, Wiley-VCH, Weinheim 2006.

Die Skalierung von Membranverfahren erfolgt im Allgemeinen durch Erhöhung der installierten Membranfläche, wie beschrieben in Melin T., Rautenbach R., Membranverfahren, Grundlagen der Modul- und Anlagenauslegung, Springer-Verlag, Berlin, Heidelberg 2007.

Die Besonderheit des beanspruchten Verfahrens liegt damit in der Kombination der oben geschilderten Reaktivdestillation K1 mit einem Reaktor und der weiteren geschickten Kombination von Reaktor und Reaktivdestillation mit konventionellen Schritten zur Destillation und Wasserabtrennung, wobei alle Schritte skalierbar sind, sowie in der geschickten Verwertung von Rückführströmen im OME-Verfahren selbst oder in externen Verfahren wie der Formaldehydherstellung.

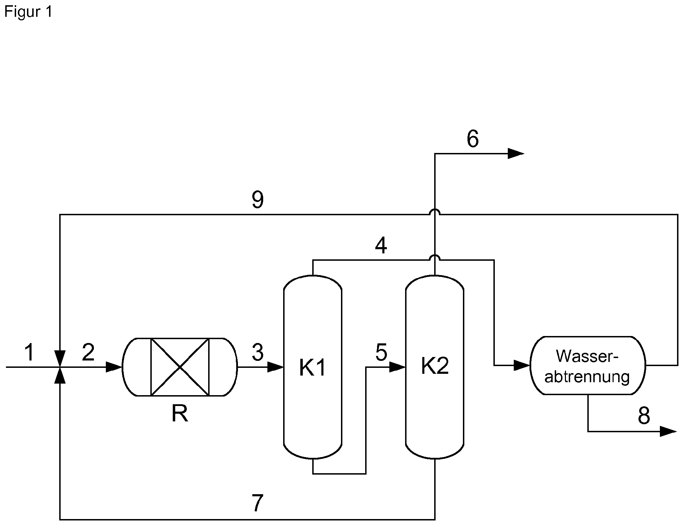

Eine detaillierte Skizze einer bevorzugten Ausführungsform des beanspruchten Verfahrens ist in Figur 1 gezeigt.

Figur 1: Skizze des beanspruchten Verfahrens. Die Abbildung zeigt eine bevorzugte Variante des erfindungsgemäßen Verfahrens.

Das Verfahren umfasst im Einzelnen die Schritte:
(i) Einspeisung von Formaldehyd und von Methanol im Allgemeinen in Form einer oder mehrerer wässriger Lösungen (Strom 1) sowie der Rückführströme 7 und 9 in einen Reaktor und Umsetzung zu einem Gemisch (Strom 3) enthaltend Formaldehyd, Wasser, Methylenglykol (MG), Polyoxymethylenglykole (MGₙ), Methanol, Hemiformale (HF), Methylal (OMEₙ₌₁) und Polyoxymethylendimethylether (OME_{n>1}); dabei ist Strom 2 eine Mischung der Ströme 1, 7 und 9. Der Zulaufstrom 1 kann geringe Mengen weiterer Komponenten (z.B. Ameisensäure, Methylformiat und weitere dem Fachmann bekannte Verunreinigungen) enthalten; Im Allgemeinen wird Formaldehyd als wässrige Formaldehyd-Lösung mit einem Formaldehyd-Gehalt von 20 bis 95 Gew.-% in den Reaktor R eingespeist. Formaldehyd kann auch hochkonzentriert, flüssig oder gasförmig oder als Paraformaldehyd in Form einer Schmelze oder als wässrige Suspension in den OME-Synthesereaktor eingespeist werden;
(ii) Einspeisung des Reaktionsgemisches (Strom 3) in die spezielle Reaktivdestillationskolonne K1 und Auftrennung in eine Leichtsiederfraktion enthaltend im Wesentlichen Formaldehyd, Wasser, Methylenglykol (MG), Polyoxymethylenglykole (MGₙ), Methanol, Hemiformale (HF), Methylal (OMEₙ₌₁) und Polyoxymethylendimethylether (OMEₙ₌₂) (Strom 4) und eine Schwersiederfraktion, im Wesentlichen bestehend aus Polyoxymethylendimethylether (OME_{n≥3}) (Strom 5);
(iii) Einspeisung der Schwersiederfraktion (Strom 5) in die Destillationskolonne K2 und Auftrennung in eine Leichtsiederfraktion (Produktfraktion), enthaltend die gewünschten Produkt-Polyoxymethylendimethylether OMEₙ₌₃₋₆ (Strom 6) und eine Schwersiederfraktion, enthaltend Polyoxymethylendimethylether (OME_{n>6}) (Strom 7);
(iv) Rückführung der Schwersiederfraktion (Strom 7) in den Reaktor;
(v) Einspeisung der Leichtsiederfraktion (Strom 5) in einen Apparat zur Wasserabtrennung (bevorzugt Pervaporation, Dampfpermeation und Adsorption) und Auftrennung in einen wasserarmen Strom 9 und einen wasserreichen Strom 8;
(vi) Rückführung des wasserarmen Stroms 9 in den Reaktor.

Der wasserreiche Strom 8 kann aus dem Verfahren ausgeschleust und optional in eine Formaldehyd-Anlage zurückgeführt werden.

Der Begriff "Leichtsiederfraktion" wird für das im oberen Teil, der Begriff "Schwersiederfraktion" für das im unteren Teil der Kolonne entnommene Gemisch verwendet. Im Allgemeinen wird die Leichtsiederfraktion am Kolonnenkopf, die Schwersiederfraktion am Kolonnensumpf entnommen. Dies ist jedoch nicht zwingend. Möglich ist auch die Entnahme über Seitenabzüge im Abtriebs- bzw. Verstärkungsteil der Kolonne.

"Im Wesentlichen bestehend aus" hat hier und nachfolgend die Bedeutung, dass die betreffende Fraktion zu mindestens 90 Gew.-%, vorzugsweise zu mindestens 95 Gew.- % aus den genannten Komponenten besteht.

Die Reaktion von Formaldehyd mit Methanol zu OME erfolgt nach der Bruttoreaktionsgleichung (11).

nCH₂O + 2CH₃OH ↔ CH₃ - O - (CH₂-O)n - CH₃ + H₂O (Reaktion 11)

Als feste Katalysatoren werden z.B. eingesetzt lonentauscherharze, Zeolithe, Aluminosilikate, Aluminiumdioxid, Titandioxid. Bevorzugt sind lonentauscherharze, besonders bevorzugt sind lonentauscherharze, deren Grundgerüst aus sulfonierten Polystryrol besteht (z.B. Amberlyst® 15, Amberlyst® 46). Im Allgemeinen kommen jedoch alle festen Katalysatoren in Frage, die ein acides Zentrum besitzen. Die Pseudoverweilzeit der Reaktionsmischung am Katalysator beträgt zwischen 1 s und 7200 s, bevorzugt zwischen 5 s und 3600 s, besonders bevorzugt zwischen 8 s und 1800 s. Die Pseudoverweilzeit ist hier definiert als Masse des Katalysators geteilt durch den Massenstrom des Fluides, das durch den Reaktor fließt. Die Umsetzung kann in jedem Apparat erfolgen, der zur Durchführung von Reaktionen fluider Medien an einem Festbettkontakt geeignet ist: Sie kann z.B. in Suspensionsfahrweise in einem Rührkesselreaktor (CSTR), einem Rohrreaktor oder einem Loop-Reaktor durchgeführt werden. Im weniger vorteilhaften Fall kann auch eine Reaktivdestillation eingesetzt werden. Bevorzugt ist ein Festbettreaktor, z.B. eine einfache durchströmte Katalysatorschüttung wie für die OME Synthese aus Trioxan und Methylal, beschrieben in Burger J., Ströfer E., Hasse H.: Production process for diesel fuel components poly(oxymethylene) dimethyl ethers from methane-based products by hierarchical optimization with varying model depth. Chemical Engineering Research and Design. 2013, 91(12), 2648-2662, ein Hordenreaktor oder ein Rohrbündelreaktor oder andere dem Fachmann bekannte Einrichtungen. Die Temperaturführung im Reaktor folgt nach dem Fachmann bekannten Regeln. Sie ist aufgrund der vergleichsweise geringen Wärmetönung standardmäßig auszuführen. Der Begriff "Reaktor" kann einen oder mehrere Apparate in Parallel- oder Seriell-Bauweise umfassen.

Das Produktgemisch kann anschließend mit einem Anionenaustauscherharz in Kontakt gebracht werden, um ein im Wesentlichen säurefreies Produktgemisch zu erhalten.

Die Umsetzung erfolgt im Allgemeinen bei einer Temperatur von 0 bis 200°C, bevorzugt 30 bis 150°C, besonders bevorzugt 40 bis 120°C und einem Druck von 0,3 bis 30 bar, bevorzugt 1 bis 20 bar, ganz besonders bevorzugt 1,2 bis 10 bar. Es ist auch möglich, die Edukte einem Festbettreaktor gasförmig zuzuführen. In diesem Falle müssen flüssige Edukte vor Eintritt in den Reaktor verdampft werden. Alternativ können gasförmige Eduktströme aus einer vorgeschalteten Produktionseinheit ohne Zwischenkondensation und nach einer eventuellen Zwischentemperierung direkt dem Reaktor zugeführt werden. Solche gasförmigen Ströme können z.B. ein gasförmiger, Formaldehyd und Wasser enthaltender Reaktoraustragstrom aus einer Formaldehyd-Anlage sein. Werden die Edukte dem Festbettreaktor gasförmig zugeführt, so erfolgt vor oder in der destillativen Aufarbeitung eine Kondensation oder Teilkondensation des Reaktoraustrags. Diese Kondensation oder Teilkondensation kann auch schon im Reaktor selbst erfolgen, wenn durch die Temperaturführung im Reaktor der Taupunkt von gebildeten Oligomeren unterschritten wird.

Die flüssige Reaktionsmischung bildet auch ohne Zugabe eines Katalysators Polyoxymethylenglykole und Polyoxymethylenhemiformale als Koppelprodukte. Die bei der Bildung der Polyoxymethylenglykole, Polyoxymethylenhemiformale und OME involvierten Kondensations- bzw. Kettenaufbaureaktionen sind Gleichgewichtsreaktionen und laufen daher (je nach Lage des chemischen Gleichgewichts) als Spaltungs- bzw. Kettenabbaureaktionen auch in umgekehrter Richtung ab. Daraus ergeben sich die besonderen Anforderungen an die Reaktivdestillationskolonne K1.

Da die chemischen Reaktionen in der Kolonne K1 mit endlicher Reaktionsgeschwindigkeit ablaufen, ist die Wahl geeigneter Kolonneneinbauten von wichtiger Bedeutung. Aufgrund der endlichen Reaktionsgeschwindigkeit der chemischen Reaktionen werden bevorzugt solche Einbauten verwendet, die eine hohe Verweilzeit und einen hohen Flüssigkeitsinhalt des Gemisches in der Kolonne ermöglichen. Werden strukturierte Packungen verwendet, kommen insbesondere spezielle Anstaupackungen in Betracht, wie sie in EP 1 074 296 beschrieben sind. Werden Böden verwendet, kommen insbesondere. spezielle Verweilzeitböden in Betracht (z.B. Thormann®-Böden). Zur Erhöhung der Verweilzeit ist es ebenfalls denkbar, die Kolonne K1 mit einem Seitenreaktor oder mehreren Seitenreaktoren auszurüsten. Dabei wird der Kolonne für jeden Seitenreaktor ein Seitenabzug entnommen, der in einen Seitenbehälter geleitet wird, in dem die chemischen Reaktionen zusätzlich ablaufen können. Der Ablaufstrom aus dem Seitenbehälter wird in die Kolonne zurückgefahren.

Je höher die Konzentration von Formaldehyd im Zulauf der Kolonne ist, desto höher wird vorzugsweise der Druck der Kolonne gewählt, um einen Feststoffausfall zu vermeiden. Bis ca. 5 Gew.-% Formaldehyd wird die Kolonne bevorzugt bei 1 bar betrieben. Ab ca. 5 Gew.-% Formaldehyd wird die Kolonne bevorzugt als Druckkolonne betrieben.

Außer der Kolonne K1 sind die weiteren Destillationskolonnen im Allgemeinen Kolonnen üblicher Bauart. In Frage kommen Füllkörperkolonnen, Bodenkolonnen und Packungskolonnen und Kombinationen, bevorzugt sind Bodenkolonnen und Packungskolonnen.

Die Destillationskolonne K2 wird bei einem Druck von 0,01 bis 1 bar, bevorzugt bei einem Druck von 0,05 bis 0,9 bar und besonders bevorzugt bei einem Druck von 0,1 bis 0.5 bar betrieben. Die Betriebstemperaturen liegen im Allgemeinen bei 60 bis 300°C. Die Kolonne weist eine theoretische Stufenzahl von 5 bis 40, bevorzugt von 8 bis 20, besonders bevorzugt von 10 bis 18 auf. Die Auslegung der Kolonne K2 erfolgt standardmäßig nach den dem Fachmann bekannten Regeln. Die Trennung in der Kolonne K2 ist in der Literatur beschrieben (Burger J., Ströfer E., Hasse H. Production process for diesel fuel components poly(oxymethylene) dimethyl ethers from methane-based products by hierarchical optimization with varying model depth. Chemical Engineering Research and Design. 2013, 91(12), 2648-2662)

Da die gewünschten OME der Kettenlänge n = 3-6 eine mittelsiedende Fraktion des Reaktoraustrags darstellen, ist es zur Erhöhung der Effizienz des Gesamtprozesses ebenfalls denkbar, die beiden Kolonnen K1 und K2 in einer einzigen reaktiven Trennwandkolonne zu integrieren. Dadurch sinken sowohl die Investitions- als auch die Betriebskosten der Anlage. Solche Trennwandkolonnen sind z.B. in US 5,914,012 beschrieben.

In diesem Fall wird in die Kolonne eine Trennwand eingebaut, die sich über den Abtriebsteil, den Bereich des Zulaufstroms und zumindest einen Teil des Verstärkerteils der Kolonne durchgängig erstreckt. Sie kann sich jedoch auch über den gesamten Verstärkerteil mit erstrecken. In dieser reaktiven Trennwandkolonne fällt als Kopfprodukt eine Mischung aus Formaldehyd, Methanol, Wasser, Methylal und OMEₙ₌₂ an. Das Seitenprodukt, welches in Höhe des Zulaufs oder zwischen Zulauf und Sumpf auf der dem Zulauf gegenüberliegenden Seite der Trennwand der Kolonne entnommen wird, ist der gewünschte Produktstrom enthaltend OME der Kettenlänge n = 3-6. Im Sumpf der Kolonne fallen die OME der Kettenlängen n ≥ 6 an. Diese reaktive Trennwandkolonne wird bei einem Druck von 0,01 bis 5 bar, bevorzugt bei einem Druck von 0,05 bis 4 bar und besonders bevorzugt bei einem Druck von 0,01 bis 3 bar betrieben. Die Betriebstemperaturen sind im Wesentlichen analog zu den Temperaturen, wenn die Kolonnen K1 und K2 einzeln betrieben werden. Die Kolonne weist eine theoretische Stufenzahl von 15 bis 80, bevorzugt 30 bis 50, besonders bevorzugt 35 bis 40 auf. Dabei entfallen 10 bis 30, vorzugsweise 15 bis 25 Stufen auf den Bereich mit eingebauter Trennwand.

Zur Erhöhung der Energieeffizienz des Prozesses ist es möglich, die Destillationskolonnen thermisch zu koppeln. Dabei wird durch geeignete Temperatur- und Druckführung der Kolonnen z.B. der Brüden einer Kolonne zum Heizen einer anderen Kolonne verwendet. Es ist ebenfalls denkbar, die Brüden der Kolonnen zu komprimieren und als Heizmedium für verschiedene Verdampfer des Prozesses zu nutzen. Es ist ebenfalls denkbar, die Anlage energetisch mit anderen Anlagen im Verbund zu koppeln, bevorzugt mit einer Formaldehyd-Anlage. Die abgeführte Reaktionswärme aus dem Formaldehyd-Reaktor kann beispielsweise zur Erzeugung von Heizdampf für die OME-Anlage verwendet werden.

Strom 6 stellt das Wertprodukt des erfindungsgemäßen Verfahrens dar. Er enthält mehr als 95 Gew.-% OMEₙ₌₃₋₆, bevorzugt mehr als 98 Gew.-% OMEₙ₌₃₋₆, ganz besonders bevorzugt mehr als 99% OMEₙ₌₃₋₆.

Für den Fall der Ausgestaltung der Wasserabtrennung als Pervaporations- oder Dampfpermeationsanlage können anorganische und polymere Membranen eingesetzt werden. Als polymere Membranen kommen z.B. in Betracht: Polyamid, Polyamidimid, Polyacrylnitril, Polybenzimidazol, Polyester, Polycarbonat, Polyetherimid, Polyethylenimin, Polyimid, Polymethylmethacrylat, Polypropylen, Polysulfon, Polyethersulfon, Polyphenylsulfon, Polytetrafluorethylen, Polyvinylidenfluorid, Polyvinylpyrrolidon, Polyvinylalkohol, Polydimethylsiloxan. Der Grad der Quervernetzung der polymeren Membran ist dabei frei einstellbar. Die mechanische Stabilität der Membranen kann durch eine Stützschicht erhöht werden. Als anorganische Membran kommen z.B. in Betracht: Zeolithe, Metalloxide wie z.B. Aluminiumdioxid, Zirkondioxid, Siliziumdioxid, Titanoxid und Glas. Die Porenstruktur der Membranen kann sowohl symmetrisch als auch asymmetrisch sein. Bevorzugt werden anorganische Membranen eingesetzt, besonders bevorzugt werden anorganische Zeolithe als Membran verwendet. Die Anordnung der Membranmodule erfolgt bevorzugt als, ist aber nicht beschränkt auf, eine "Tannenbaumstruktur". Dadurch werden alle eingesetzten Membranmodule fluiddynamisch gleich belastet. Als Membranmodule kommen alle dem Fachmann bekannten Modulformen wie z.B. Rohrmodule, Plattenmodule, Kapillarmodule und Hohlfasermodule in Betracht. Falls polymere Membranen eingesetzt werden, sind Plattenmodule bevorzugt. Falls anorganische Membranen verwendet werden, sind Rohrmodule bevorzugt.

Der Betriebsdruck auf der Zulaufseite der Membran beträgt zwischen 0,1 und 200 bar, bevorzugt zwischen 1 und 150 bar. Der Druck auf der Permeatseite beträgt zwischen 0,001 und 10 bar, bevorzugt zwischen 0,01 und 5 bar. Die Temperatur beträgt zwischen 30 und 200°C. Dem Fachmann ist bekannt, dass insbesondere für den Fall der Pervaporation zwischen den Membranmodulen im Allgemeinen eine Erwärmung der Retentatströme durch zwischengeschaltete Wärmeüberträger erfolgt. Insbesondere ist es möglich, den Retentatstrom aus dem letzten Membranmodul zur Vorheizung des Zulaufs des ersten Membranmoduls zu verwenden.

Je nach gewähltem Druckniveau auf der Permeatseite der Membran erfolgt eine Kondensation des Permeatstroms durch Kühlwasser oder Kühlsole. Andere Prozessströme aus der OME-Anlage oder einer Formaldehyd-Anlage können ebenfalls eingesetzt werden. Kühlsole wird bevorzugt bei sehr niedrigen Drücken auf der Permeatseite eingesetzt. Wird auf der Permeatseite ein Vakuum angelegt, kann sowohl eine mechanische als auch eine thermisch arbeitende Vakuumpumpe verwendet werden. Im letzteren Fall wird bevorzugt ein Strahlverdichter verwendet.

Wird eine Dampfpermeationsanlage verwendet, so ist es möglich, die Kolonne K1 nur mit einem Partialkondensator zu betreiben, so dass der nicht kondensierte Brüden der Kolonne K1 direkt in die Dampfpermeationsanlage geleitet wird. Es ist jedoch weiterhin auch möglich, die Kolonne K1 mit einem Totalkondensator zu betreiben und den Zulaufstrom der Dampfpermeationsanlage vorher mit Hilfe eines zusätzlichen Wärmeübertragers zu verdampfen.

Für den Fall der Ausgestaltung der Wasserabtrennung als Adsorptionsanlage sind alle gängigen Adsorbermaterialen einsetzbar. Beispiele sind Zeolithe, Aktivkohle, Adsorberharze, Metalloxide wie z.B. Aluminiumdioxid, Zirkondioxid, Siliziumdioxid (Kieselgele, Silikagele) und Titanoxid, Salze wie z.B. Magnesiumsulfat, Natriumsulfat, Calciumhydrid, Calciumoxid, Calciumsulfat, Kaliumcarbonat, Kaliumhydroxid, Kupfersulfat, Lithiumaluminiumhydrid, Natriumhydroxid, und elementare Alkali- und Erdalkalimetalle. Bevorzugt werden Zeolithe und Metalloxide verwendet, ganz besonders bevorzugt werden Zeolithe verwendet. Die bevorzugten Adsorbermaterialien können dabei besonders einfach durch geeignete Temperatur- und Druckführung regeneriert werden. Werden Zeolithe als Adsorptionsmittel eingesetzt, so werden bevorzugt Zeolithe von Typ NaA eingesetzt, besonders bevorzugt Zeolithe von Typ NaA, deren Porenweite zwischen 2 und 10 Angström beträgt. Der wasserreiche Strom F6 fällt im diskontinuierlichen Betrieb bei der Regeneration des Adsorbers an. Dabei desorbieren Wasser und die zusätzlich adsorbierten Komponenten.

Die Adsorptionsanlage wird bevorzugt als einfache Festbettanlage ausgeführt. Möglich sind jedoch auch alle weiteren, dem Fachmann bekannten Bauarten von Adsorbern. Die Regeneration des Adsorbermaterials erfolgt in diesem Fall bevorzugt durch Druck- und/oder Temperaturwechsel im diskontinuierlichen Betrieb. Daher werden im Regelfall mindestens zwei Adsorber-Festbetten benötigt, so dass während des Betriebs eines Festbetts das andere Festbett regeneriert werden kann. Üblich ist eine Anordnung mit mehr ≥ 3 Adsorberbetten im Parallelbetrieb. Der Zulaufstrom zum Adsorber kann sowohl gasförmig als auch flüssig sein.

Es ist auch denkbar, den Strom 4 ohne Wasserabtrennung direkt in eine Formaldehyd-Anlage zurückzuführen und die Wasserabtrennung in der Formaldehyd-Anlage durchzuführen. Dabei reagieren neben Methanol auch Vollacetale aus Strom 4 im Reaktor der Formaldehyd-Synthese zu Formaldehyd und Wasser (Masamoto J., Matsuzaki K., Development of methylal synthesis by reactive destillation; Journal of Chemical Engineering of Japan, 1994, 27(1), 1-5). Die Wasserabtrennung ist durch eine anschließende Aufkonzentrierung der erhaltenen Formaldehyd Lösung möglich, wie sie z.B. in US 7,342,139; US 7,345,207; US 7,273,955; US 7,193,115; US 6,610,888; US 6,610,888; US 7,414,159 beschrieben sind. Die aufkonzentrierte Lösung wird wieder für die OME-Synthese im Reaktor der OME-Synthese bereitgestellt. Auch kann, wie in US 7,390,932 beschrieben, hochkonzentriertes Formaldehyd in der Gasphase hergestellt werden und dem Reaktor zugeführt werden. Ferner kann die wässrige Formaldehydlösung nach bekannten Verfahren in Paraformaldehyd überführt werden, der als Schmelze oder Feststoff-Suspension dem Reaktor zugeführt wird. Auch unabhängig von der Rückführung des Stromes 4 können hochkonzentriertes flüssiges oder gasförmiges Formaldehyd oder Paraformaldehyd als Schmelze oder Suspension im OME-Synthesereaktor eingesetzt werden.

Neben dem bevorzugten Einsatzstoff Methanol können in der OME-Synthese als Reaktionspartner des Formaldehyds Derivate des Methanols eingesetzt werden wie z.B. Methylal, Dimethylether, OME₂ und/oder Mischungen derselben untereinander und/oder mit Methanol.

Neben dem Methanol als einfachstem Alkohol und seinen Derivaten können als Reaktionspartner des Formaldehyds im Prinzip auch andere Alkohole eingesetzt werden, wie z.B. Ethanol, Propanol und Butanol und/oder Mischungen derselben und/oder mit Methanol. Was oben über die Derivate des Methanols gesagt wurde gilt analog für die Derivate anderer Alkohole, wobei auch Mischderivate wie z.B. C₂H₅O - CH₂ - OCH₃ oder C₂H₅-O-CH₃ möglich sind. Im Falle von Mischungslücken werden Emulsionen eingesetzt.

Das erfindungsgemäße Verfahren kann ganz oder teilweise kontinuierlich oder absatzweise betrieben werden. Dies gilt auch für den Reaktor und die Kolonnen. Bevorzugt ist der kontinuierliche Betrieb des Verfahrens. Einheiten, wie z.B. die Wasserabtrennung durch Adsorption, die technisch generell bevorzugt absatzweise betrieben werden, werden dabei in mehrere Apparaten in Parallelbauweise so gestaltet, dass praktisch ein kontinuierlicher Fluss aller Ströme durch diese Einheit möglich ist.

Gegenstand der Erfindung sind weiterhin die nach dem erfindungsgemäßen Verfahren erhältlichen Produkte sowie die Abmischungen dieser Produkte mit Kraftstoff- und Ölfraktionen aus einer Raffinerie oder einem Raffinerieverbund. Diese Abmischungen können ferner Kraftstoffhilfsstoffe und Additive enthalten, wie sie dem Kraftstofffachmann bekannt sind.

Die Zusammensetzung eines Gemischs von OME-Oligomeren ist, wenn der Reaktor des Verfahrens nahe am Punkt des Gleichgewichtsumsatzes betrieben wird: 0,35 g/g ≤ x_{OME3} ≤ 0,79 g/g, 0,17 g/g ≤ x_{OME4} ≤ 0,36 g/g, 0,04 g/g ≤ x_{OMES} ≤ 0,31 g/g, x_{OME6} ≤ 0,06 g/g, bevorzugt 0,37 g/g ≤ x_{OME3} ≤ 0,70 g/g; 0,23 g/g ≤ x_{OME4} ≤ 0,35 g/g, 0,07 g/g ≤ x_{OME5} ≤0,26 g/g, x_{OME6} ≤ 0,08 g/g, ganz besonders bevorzugt 0,40 g/g ≤ x_{OME3} ≤ 0,62 g/g; 0,26 g/g ≤ x_{OME4} ≤ 0,35 g/g, 0,11 g/g ≤ x_{OME5} ≤ 0,26 g/g, x_{OME6} ≤ 0,12 g/g. Dabei handelt es sich bei x_{OMEn} um den Massenanteil des OME-Oligomeren mit n Oxymethylen-Einheiten, bezogen auf die Masse des Gemischs.

"Nahe am Gleichgewicht" bedeutet hier, dass keine Konzentration der oben genannten einzelnen OME-Spezies sich mehr als 40% entfernt von dem Wert befindet, der der Zusammensetzung eines Reaktionsgemisches entspricht, welches sich im thermodynamischen Gleichgewicht befindet.

Die Zusammensetzung dieser Mischung von OME-Oligomeren ist eine Folge der oben beschriebenen Verfahrensführung.

Je nach Kundennachfrage können jedoch auch andere Mischungen von OME-Oligomeren hergestellt werden. Diese Herstellung erfolgt durch Variation der Verfahrensführung im Reaktor und besonders der Lage der Trennschnitte in den Kolonnen. Die Rückführströme müssen dann entsprechend angepasst werden und das Verfahren arbeitet nicht mehr am ökonomischen und ökologischen Optimum.

Eine weitere Auftrennung des Produktes OMEₙ₌₃₋₆ nach der Kettenlänge ist durch verfahrenstechnische Operationen, wie zum Beispiel eine Destillation, möglich. Die Auslegung einer Destillationssequenz zur Auftrennung von OMEₙ₌₃₋₆ nach der Kettenlänge ist in diesem Fall standardmäßig auszuführen. Bevorzugt (jedoch nicht ausschließlich) wird zunächst OME₃ als Kopfprodukt einer ersten Destillationskolonne, OME₄ als Kopfprodukt einer zweiten Destillationskolonne, OME₅ als Kopfprodukt einer dritten Destillationskolonne und OME₆ als Sumpfprodukt der dritten Destillationskolonne gewonnen. Die optionale Auftrennung des Produktes OMEₙ₌₃₋₆ ändert jedoch nichts an der oben beschriebenen Verteilung der OME-Oligomeren untereinander, da die Verhältnisse der OME-Kettenlängen durch die Auftrennung nur unwesentlich durch die in der Realität nicht perfekt scharfen Trennschnitte beeinflusst werden.

Die Erfindung wird durch die nachfolgenden Beispiele näher erläutert.

### Beispiele

### Beispiel 1: Gesamtverfahren

In Tabelle 1 bis Tabelle 4 sind vier typische Stromleisten einer bevorzugten Variante des beanspruchten Verfahrens innerhalb der Messgenauigkeiten dargestellt. Die Zahlen sind entsprechend gerundet. Dabei bedeutet der Massenanteil 0,00 g/g einen Massenanteil < 0,005 g/g. In Tabelle 1 bis Tabelle 3 beträgt das Massenverhältnis Formaldehyd zu Methanol in Strom 1, 1,65. Der Wasseranteil in Strom 1 wurde variiert zwischen 0,05 g/g (Tabelle 1), 0,10 g/g (Tabelle 2) und 0,20 g/g (Tabelle 3). In Tabelle 4 beträgt das Massenverhältnis Formaldehyd zu Methanol in Strom 1, 1,60. Der Wasseranteil in Strom 1 ist 0,10 g/g.

Die Wasserabtrennung wurde dabei in allen Fällen so dimensioniert, dass der Wasseranteil in Strom 9 ca. 0,02 g/g beträgt. Da der Wasseranteil in Strom 1 von Tabelle 1 bis Tabelle 3 erhöht wurde, ist, damit der Wassermassenanteil in Strom 9 konstant ist, im Allgemeinen für den Fall der Ausgestaltung der Wasserabtrennung als Dampfpermeations- oder Pervaporationsanlage die installierte Membranfläche erhöht worden und für den Fall der Ausgestaltung der Wasserabtrennung als Adsorptionsanlage die Adsorbermasse erhöht worden.

Aufgrund der Durchführung der Versuche im Mikromaßstab ist jedoch in diesem Beispiel die Erhöhung der Membranfläche vernachlässigbar klein.

Der Massenstrom und die Zusammensetzung von Strom 8 (ausgeschleuster Wasserstrom) kann je nach Art der Ausgestaltung der Wasserabtrennung variieren. In den Tabellen ergibt sich Strom 8 (nicht angegeben) aus der Differenz der Ströme 4 und 9.

**Tabelle 1: Beispiel (Fall 1) für eine Stromleiste des erfindungsgemäßen Verfahrens; Zulaufzusammensetzung (Strom 1): Wasseranteil = 0,05 g/g, Massenverhältnis Formaldehyd zu Methanol = 1,65**

| Strom | 1 | 2 | 3 | 4 | 5 | 6 | 7 | 9 |
|---|---|---|---|---|---|---|---|---|
| Massenstrom / (kg/h) | 1,2 | 6,1 | 6,1 | 5,0 | 1,1 | 1,0 | 0,1 | 4,8 |

| Massenanteile / (g/g) | | | | | | | | |
|---|---|---|---|---|---|---|---|---|
| Formaldehyd | 0,59 | 0,36 | 0,25 | 0,30 | 0,00 | 0,00 | 0,00 | 0,32 |
| Methanol | 0,36 | 0,20 | 0,13 | 0,16 | 0,00 | 0,00 | 0,00 | 0,17 |
| Wasser | 0,05 | 0,03 | 0,04 | 0,05 | 0,00 | 0,00 | 0,00 | 0,02 |
| Methylal | 0,00 | 0,24 | 0,24 | 0,29 | 0,00 | 0,00 | 0,00 | 0,30 |
| OME₂ | 0,00 | 0,15 | 0,15 | 0,19 | 0,00 | <0,02 | 0,00 | 0,19 |
| OME₃ | 0,00 | 0,00 | 0,09 | <0,01 | 0,47 | 0,54 | 0,00 | 0,00 |
| OME₄ | 0,00 | 0,00 | 0,05 | 0,00 | 0,26 | 0,30 | 0,00 | 0,00 |
| OME₅ | 0,00 | 0,00 | 0,03 | 0,00 | 0,14 | 0,16 | 0,00 | 0,00 |
| OME₆ | 0,00 | 0,01 | 0,01 | 0,00 | 0,07 | <0,02 | 0,56 | 0,00 |
| OME₇ | 0,00 | 0,01 | 0,01 | 0,00 | 0,04 | 0,00 | 0,29 | 0,00 |
| OME₈ | 0,00 | 0,00 | 0,00 | 0,00 | 0,02 | 0,00 | 0,15 | 0,00 |

Für die Stromleiste aus Tabelle 1 (Fall 1) wurde eine Versuchsanlage im Mikromaßstab betrieben. Als Reaktor wurde ein Festbettreaktor mit 200 g des heterogenen Katalysators Amberlyst® 46 verwendet, der vorher mit Methanol gespült wurde. Der Reaktor wurde bei 60°C betrieben. Die Reaktivdestillationskolonne K1 wurde mit der strukturierten Packung Sulzer CY mit 20 theoretischen Trennstufen ausgestattet. Der Zulauf erfolgte auf Stufe auf Stufe 10. Der Druck in der Kolonne K1 betrug 2 bar. Die Kopftemperatur in der Kolonne K1 betrug 85°C, die Sumpftemperatur betrug 200°C. Die Kolonne K1 wurde mit einem Rücklaufverhältnis von 1,0 kg/kg betrieben. Die Zulauftemperatur der Kolonne K1 entsprach der Reaktortemperatur von 60°C. Die Kolonne K2 wurde mit der strukturierten Laborpackung Sulzer DX mit 15 theoretischen Trennstufen ausgestattet. Der Zulauf erfolgte auf Stufe 8 mit einer Temperatur von ebenfalls 60°C. Der Druck in der Kolonne K2 betrug 0,1 bar. Die Kopftemperatur in der Kolonne K2 betrug 97°C, die Sumpftemperatur betrug 203°C. Die Kolonne K2 wurde mit einem Rücklaufverhältnis von 0,8 kg/kg betrieben. Die Wasserabtrennung erfolgte mittels Pervaporation. Als Membran wurde eine Membran aus NaA Zeolith der Porenweite 4,2 Å verwendet. Die effektive Membranfläche betrug ca. 2 m². Die Pervaporation erfolgte bei 70°C und einem Zulaufdruck von 2 bar. Auf der Permeatseite wurde ein Vakuum von 50 mbar eingestellt. Der Betrieb der Versuchsanlage erfolgte für 8 h im kontinuierlichen Betrieb.

**Tabelle 2: Beispiel (Fall 2) für eine Stromleiste des beanspruchten Verfahrens; Zulaufzusammensetzung (Strom 1): Wasseranteil = 0,10 g/g; Massenverhältnis Formaldehyd zu Methanol = 1,65**

| | | | | | | | | |
|---|---|---|---|---|---|---|---|---|
| Strom | 1 | 2 | 3 | 4 | 5 | 6 | 7 | 9 |
| Massenstrom / (kg/h) | 1,2 | 6,5 | 6,5 | 5,4 | 1,1 | 1,0 | 0,1 | 5,1 |

| Massenanteile / (g/g) | | | | | | | | |
|---|---|---|---|---|---|---|---|---|
| Formaldehyd | 0,56 | 0,37 | 0,27 | 0,32 | 0,00 | 0,00 | 0,00 | 0,34 |
| Methanol | 0,34 | 0,20 | 0,14 | 0,17 | 0,00 | 0,00 | 0,00 | 0,18 |
| Wasser | 0,10 | 0,03 | 0,05 | 0,06 | 0,00 | 0,00 | 0,00 | 0,02 |
| Methylal | 0,00 | 0,22 | 0,22 | 0,27 | 0,00 | 0,00 | 0,00 | 0,29 |
| OME₂ | 0,00 | 0,14 | 0,14 | 0,17 | 0,00 | <0,02 | 0,00 | 0,18 |
| OME₃ | 0,00 | 0,00 | 0,08 | <0,01 | 0,47 | 0,54 | 0,00 | 0,00 |
| OME₄ | 0,00 | 0,00 | 0,05 | 0,00 | 0,26 | 0,30 | 0,00 | 0,00 |
| OME₅ | 0,00 | 0,00 | 0,02 | 0,00 | 0,14 | 0,16 | 0,00 | 0,00 |
| OME₆ | 0,00 | 0,01 | 0,01 | 0,00 | 0,07 | <0,02 | 0,56 | 0,00 |
| OME₇ | 0,00 | 0,01 | 0,01 | 0,00 | 0,04 | 0,00 | 0,29 | 0,00 |
| OME₈ | 0,00 | 0,00 | 0,00 | 0,00 | 0,02 | 0,00 | 0,15 | 0,00 |

Für die Stromleiste aus Tabelle 2 (Fall 2) wurde eine Versuchsanlage im Mikromaßstab betrieben. Die Kopftemperatur der Reaktivdestillationskolonne K1 betrug hier 89°C, die Sumpftemperatur betrug 202°C. Die Betriebsparameter aller anderen Apparate entsprechen dabei im Rahmen der Messungenauigkeiten denen aus Fall 1. Insbesondere sind die Kopf- und Sumpftemperaturen für die Kolonne K2 aufgrund der ähnlichen Zusammensetzung des Zulaufstroms 5 gleich.

**Tabelle 3: Beispiel (Fall 3) für eine Stromleiste des beanspruchten Verfahrens; Zulaufzusammensetzung (Strom 1): Wasseranteil = 0,20 g/g; Massenverhältnis Formaldehyd zu Methanol = 1,65**

| | | | | | | | | |
|---|---|---|---|---|---|---|---|---|
| Strom | 1 | 2 | 3 | 4 | 5 | 6 | 7 | 9 |
| Massenstrom / (kg/h) | 1,4 | 7,3 | 7,3 | 6,2 | 1,1 | 1,0 | 0,1 | 5,8 |

| Massenanteile / (g/g) | | | | | | | | |
|---|---|---|---|---|---|---|---|---|
| Formaldehyd | 0,50 | 0,39 | 0,30 | 0,35 | 0,00 | 0,00 | 0,00 | 0,38 |
| Methanol | 0,30 | 0,21 | 0,15 | 0,18 | 0,00 | 0,00 | 0,00 | 0,19 |
| Wasser | 0,20 | 0,05 | 0,07 | 0,08 | 0,00 | 0,00 | 0,00 | 0,02 |
| Methylal | 0,00 | 0,20 | 0,20 | 0,24 | 0,00 | 0,00 | 0,00 | 0,25 |
| OME₂ | 0,00 | 0,13 | 0,13 | 0,15 | 0,00 | <0,02 | 0,00 | 0,16 |
| OME₃ | 0,00 | 0,00 | 0,07 | <0,01 | 0,47 | 0,54 | 0,00 | 0,00 |
| OME₄ | 0,00 | 0,00 | 0,04 | 0,00 | 0,26 | 0,30 | 0,00 | 0,00 |
| OME₅ | 0,00 | 0,00 | 0,02 | 0,00 | 0,14 | 0,16 | 0,00 | 0,00 |
| OME₆ | 0,00 | 0,01 | 0,01 | 0,00 | 0,07 | <0,02 | 0,56 | 0,00 |
| OME₇ | 0,00 | 0,01 | 0,01 | 0,00 | 0,04 | 0,00 | 0,29 | 0,00 |
| OME₈ | 0,00 | 0,00 | 0,00 | 0,00 | 0,02 | 0,00 | 0,15 | 0,00 |

Für die Stromleiste aus Tabelle 3 (Fall 3) wurde eine Versuchsanlage im Mikromaßstab betrieben. Die Kopftemperatur der Reaktivdestillationskolonne K1 betrug hier 91 °C, die Sumpftemperatur betrug 202°C. Die Betriebsparameter aller anderen Apparate entsprechen dabei im Rahmen der Messungenauigkeiten denen aus Fall 1. Insbesondere sind die Kopf- und Sumpftemperaturen für die Kolonne K2 aufgrund der ähnlichen Zusammensetzung des Zulaufstroms 5 gleich.

**Tabelle 4: Beispiel (Fall 4) für eine Stromleiste des beanspruchten Verfahrens; Zulaufzusammensetzung (Strom 1): Wasseranteil = 0,1 g/g; Massenverhältnis Formaldehyd zu Methanol = 1,60**

| | | | | | | | | |
|---|---|---|---|---|---|---|---|---|
| Strom | 1 | 2 | 3 | 4 | 5 | 6 | 7 | 9 |
| Massenstrom / (kg/h) | 1,2 | 8,0 | 8,0 | 6,9 | 1,1 | 1,0 | 0,1 | 6,7 |

| Massenanteile / (g/g) | | | | | | | | |
|---|---|---|---|---|---|---|---|---|
| Formaldehyd | 0,55 | 0,27 | 0,19 | 0,22 | 0,00 | 0,00 | 0,00 | 0,22 |
| Methanol | 0,35 | 0,18 | 0,13 | 0,15 | 0,00 | 0,00 | 0,00 | 0,16 |
| Wasser | 0,10 | 0,03 | 0,05 | 0,05 | 0,00 | 0,00 | 0,00 | 0,02 |
| Methylal | 0,00 | 0,33 | 0,33 | 0,39 | 0,00 | 0,00 | 0,00 | 0,40 |
| OME₂ | 0,00 | 0,17 | 0,17 | 0,19 | 0,00 | <0,02 | 0,00 | 0,20 |
| OME₃ | 0,00 | 0,00 | 0,08 | <0,01 | 0,57 | 0,62 | 0,00 | 0,00 |
| OME₄ | 0,00 | 0,00 | 0,03 | 0,00 | 0,25 | 0,27 | 0,00 | 0,00 |
| OME₅ | 0,00 | 0,00 | 0,01 | 0,00 | 0,11 | 0,11 | 0,00 | 0,00 |
| OME₆ | 0,00 | 0,01 | 0,01 | 0,00 | 0,04 | <0,02 | 0,64 | 0,00 |
| OME₇ | 0,00 | 0,00 | 0,00 | 0,00 | 0,02 | 0,00 | 0,26 | 0,00 |
| OME₈ | 0,00 | 0,00 | 0,00 | 0,00 | 0,01 | 0,00 | 0,10 | 0,00 |

Für die Stromleiste aus Tabelle 4 (Fall 4) wurde eine Versuchsanlage im Mikromaßstab betrieben. Die Kopftemperatur in der Kolonne K1 betrug hier 82°C, die Sumpftemperatur betrug 195°C. Die Kopftemperatur in der Kolonne K2 betrug hier 94°C, die Sumpftemperatur betrug 200°C. Die Betriebsparameter aller anderen Apparate entsprechen dabei im Rahmen der Messungenauigkeiten denen aus Fall 1.

### Beispiel 2: Betrieb der speziellen Reaktivdestillationskolonne K1

Die Betriebsparameter einer Ausführung der speziellen Reaktivdestillationskolonne K1 sind in Tabelle 5 zusammengefasst. Dabei bedeutet der Massenanteil 0.00 g/g einen Massenanteil < 0,005 g/g. Die Kolonne wurde hierbei mit einem Zulauf mit der Zusammensetzung des Stroms 3 aus Tabelle 2 auf Stufe 12 beschickt. Für diesen Zulauf beträgt die Anzahl der minimal notwendigen theoretischen Trennstufen zwischen 10 und 18, je nach gewünschter Ausbeute von OME_{n≥3} im Sumpf der Kolonne. Die Kolonne wurde bei 2 bar und einem Rücklaufverhältnis von 1,0 kg/kg betrieben, die Zahl der theoretischen Trennstufen beträgt 30 (inklusive Verdampfer und Kondensator). Als trennwirksamer Einbau wurde die strukturierte Packung Sulzer CY mit einer spezifischen Oberfläche von 700 m²/m³ verwendet. Der Durchmesser der Kolonne betrug 53 mm und der Flüssigkeitsinhalt auf jeder theoretischen Trennstufe war zwischen 10 und 80 ml Flüssigkeit. Dies entspricht zwischen 1% und 8% des Leerrohrvolumens. Zusätzlich wurden zwischen jedem Schuss spezielle Fangböden und Flüssigkeitsverteiler installiert, um eine hohe Verweilzeit des Gemisches in der Kolonne zu ermöglichen.

**Tabelle 5: Betriebsparamater einer Ausführung der speziellen Reaktivdestillationskolonne K1 bei p = 2 bar.**

| | Zulauf | Sumpfprodukt | Kopfprodukt |
|---|---|---|---|
| Temperatur /(°C) | 60 | 202 | 89 |
| Massenstrom /(kg/h) | 1,10 | 0,19 | 0,91 |

| Massenanteile / (g/g) | | | |
|---|---|---|---|
| Formaldehyd | 0,27 | < 0,01 | 0,33 |
| Methanol | 0,14 | < 0,01 | 0,17 |
| Wasser | 0,05 | < 0,01 | 0,06 |
| Methylal | 0,22 | < 0,01 | 0,27 |
| OME₂ | 0,14 | 0,00 | 0,17 |
| OME₃ | 0,08 | 0,43 | < 0,01 |
| OME₄ | 0,05 | 0,28 | 0 |
| OME₅ | 0,02 | 0,11 | 0 |
| OME₆ | 0,01 | 0,06 | 0 |
| OME₇ | 0,01 | 0,06 | 0 |
| OME₈ | 0,01 | 0,06 | 0 |

In der gleichen Kolonne wurde zusätzlich ein Destillationsversuch mit einem Zulauf mit der Zusammensetzung des Stroms 3 aus Tabelle 4 auf Stufe 12 beschickt. Dies stellt eine weitere Ausführung der speziellen Reaktivdestillationskolonne K1 dar. Für diesen Zulauf beträgt die Anzahl der minimal notwendigen theoretischen Trennstufen zwischen 5 und 13, je nach gewünschter Ausbeute von OME_{n≥3} in Sumpf der Kolonne. Die Kolonne wurde bei 1,6 bar und einem Rücklaufverhältnis von 0,9 kg/kg betrieben, die Zahl der theoretischen Trennstufen beträgt wiederum 30 (inklusive Verdampfer und Kondensator). Der Flüssigkeitsinhalt auf jeder theoretischen Trennstufe war zwischen 10 und 80 ml Flüssigkeit. Dies entspricht zwischen 1% und 8% des Leerrohrvolumens. Die Betriebsparameter dieser Kolonne sind in Tabelle 6 zusammengefasst.

In beiden Ausführungen der speziellen Reaktivdestillationskolonne kann OME_{n≥3} mit sehr hoher Ausbeute im Sumpf der Kolonne gewonnen werden.

**Tabelle 6: Betriebsparamater einer Ausführung speziellen Reaktivdestillationskolonne K1 bei p = 1,6 bar**

| | Zulauf | Sumpfprodukt | Kopfprodukt |
|---|---|---|---|
| Temperatur / (°C) | 60 | 185 | 75 |
| Massenstrom / (kg/h) | 1,20 | 0,16 | 1,04 |

| Massenanteile / (g/g) | | | |
|---|---|---|---|
| Formaldehyd | 0,19 | < 0,01 | 0,22 |
| Methanol | 0,13 | < 0,01 | 0,15 |
| Wasser | 0,05 | < 0,01 | 0,05 |
| Methylal | 0,33 | < 0,01 | 0,38 |
| OME₂ | 0,17 | 0,00 | 0,19 |
| OME₃ | 0,08 | 0,56 | < 0,01 |
| OME₄ | 0,03 | 0,26 | 0 |
| OME₅ | 0,01 | 0,11 | 0 |
| OME₆ | 0,01 | 0,05 | 0 |
| OME₇ | 0,00 | 0,02 | 0 |
| OME₈ | 0,00 | 0,01 | 0 |

### Beispiel 3: Wasserabtrennung mittels Adsorption

In einem Batch-Schüttelkolbenversuch wurden vier verschiedene flüssige Mischungen bestehend aus Formaldehyd, Wasser, Methanol, Methylal OME₂ und OME₃ mit dem Adsorber Zeolith 3A bei der Temperatur 25°C in Kontakt gebracht. Dabei wurde der Wasseranteil systematisch variiert, während die Verhältnisse aller anderen Komponenten konstant bleiben. Das Massenverhältnis von Flüssigkeit zu Adsorber beträgt 2:1. Dabei wurde die Zusammensetzung vor und nach dem Adsorptionsvorgang gemessen. Die Ergebnisse sind in Tabelle 7 (vor Adsorption) und Tabelle 8 (nach Adsorption) dargestellt. Dabei ist zu erkennen, dass der Massenanteil von Wasser durch Zugabe des Adsorbers deutlich reduziert wurde. Das Adsorbermaterial wurde anschließend bei einer Temperatur > 50°C und einem Druck < 500 mbar regeneriert und konnte erneut verwendet werden.

**Tabelle 7: Zusammensetzungen der flüssigen Mischungen vor der Adsorption.**

| Mischung | 1 | 2 | 3 | 4 |
|---|---|---|---|---|
| Massenanteile / (g/g) | | | | |
| Formaldehyd | 0.21 | 0.19 | 0.18 | 0.17 |
| Methanol | 0.29 | 0.27 | 0.25 | 0.24 |
| Wasser | 0.01 | 0.07 | 0.12 | 0.16 |
| Methylal | 0.30 | 0.28 | 0.26 | 0.25 |
| OME₂ | 0.20 | 0.19 | 0.18 | 0.17 |

**Tabelle 8: Zusammensetzungen der flüssigen Mischungen nach der Adsorption**

| Mischung | 1 | 2 | 3 | 4 |
|---|---|---|---|---|
| Massenanteile / (g/g) | | | | |
| Formaldehyd | 0.21 | 0.20 | 0.19 | 0.19 |
| Methanol | 0.27 | 0.28 | 0.27 | 0.26 |
| Wasser | 0.00 | 0.02 | 0.05 | 0.09 |
| Methylal | 0.30 | 0.28 | 0.29 | 0.27 |
| OME₂ | 0.21 | 0.21 | 0.20 | 0.19 |

### Beispiel 4: Wasserabtrennung mittels Membranverfahren

In einer Labor-Pervaporationsanlage wurde eine Membran aus NaA Zeolith der Porenweite 4,2 Å mit einem Zulaufstrom entsprechend der Zusammensetzung des Stroms 4 aus Tabelle 4 beschickt. Der Zulaufdruck betrug 2 bar, und auf der Permeatseite wurde ein Vakuum von 50 mbar eingestellt. Die Pervaporation wurde bei 80°C durchgeführt und die effektive Membranfläche beträgt 70 cm². Der Permeatstrom wurde mittels einer Kühlfalle mit flüssigem Stickstoff gekühlt und anschließend analysiert. Der Massenanteil von Wasser im Permeatstrom betrug > 0,95 g/g. Neben Wasser wurden auch Methanol, Formaldehyd und Methylal im Permeatstrom nachgewiesen.

## Patentansprüche

1. Verfahren zur Herstellung von Polyoxymethylendimethylethern mit ≥3 Oxymethylen-Einheiten (OME_{n≥3}) mit den Schritten:
(i) Einspeisung von Formaldehyd, Methanol und Wasser in einen Reaktor R und Umsetzung zu einem Reaktionsgemisch enthaltend Formaldehyd, Wasser, Methylenglykol, Polyoxymethylenglykole, Methanol, Hemiformale, Methylal (OMEₙ₌₁) und Polyoxymethylendimethylether (OME_{n>1});
(ii) Einspeisung des Reaktionsgemischs in eine Reaktivdestillationskolonne K1 und Auftrennung in eine Leichtsiederfraktion F1 enthaltend Formaldehyd, Wasser, Methylenglykol, Polyoxymethylenglykole, Methanol, Hemiformale, Methylal (OMEₙ₌₁) und Polyoxymethylendimethylether mit 2 bis 3 Oxymethylen-Einheiten (OMEₙ₌₂₋₃) und eine Schwersiederfraktion F2 enthaltend Polyoxymethylendimethylether mit mehr als zwei Oxymethylen-Einheiten (OME_{n≥3}), wobei die Schwersiederfraktion F2 mindestens 90 Gew.-% Polyoxymethylen-dimethylether mit mehr als zwei Oxymethylen-Einheiten (OME_{n≥3}) enthält.

2. Verfahren nach Anspruch 1 zur Herstellung von Polyoxymethylendimethylethern mit 3 bis 6 Oxymethylen-Einheiten (OMEₙ₌₃₋₆) mit den Schritten:
(i) Einspeisung von Formaldehyd, Methanol und Wasser in einen Reaktor R und Umsetzung zu einem Reaktionsgemisch enthaltend Formaldehyd, Wasser, Methylenglykol, Polyoxymethylenglykole, Methanol, Hemiformale, Methylal (OMEₙ₌₁) und Polyoxymethylendimethylether (OME_{n>1});
(ii) Einspeisung des Reaktionsgemischs in eine Reaktivdestillationskolonne K1 und Auftrennung in eine Leichtsiederfraktion F1 enthaltend Formaldehyd, Wasser, Methylenglykol, Polyoxymethylenglykole, Methanol, Hemiformale, Methylal (OMEₙ₌₁) und Polyoxymethylendimethylether mit 2 bis 3 Oxymethylen-Einheiten (OMEₙ₌₂₋₃) und eine Schwersiederfraktion F2 enthaltend Polyoxymethylendimethylether mit mehr als zwei Oxymethylen-Einheiten (OME_{n≥3}), wobei die Schwersiederfraktion F2 mindestens 90 Gew.-% Polyoxymethylen-dimethylether mit mehr als zwei Oxymethylen-Einheiten (OME_{n≥3}) enthält;
(iii) Einspeisung der Schwersiederfraktion F2 in eine Destillationskolonne K2 und Auftrennung in eine Produktfraktion F3 enthaltend Polyoxymethylendimethylether mit 3 bis 6 Oxymethylen-Einheiten (OMEₙ₌₃₋₆) und eine Schwersiederfraktion F4 enthaltend Polyoxymethylendimethylether mit mehr als 6 Oxymethylen-Einheiten (OME_{n>6});
(iv) gegebenenfalls Rückführung der Schwersiederfraktion F4 in den Reaktor R.

3. Verfahren nach Anspruch 2, **dadurch gekennzeichnet, dass** die Schwersiederfraktion F2 mindestens 95 Gew.-% Polyoxymethylendimethylether mit mehr als zwei Oxymethylen-Einheiten (OME_{n≥3}) enthält.

4. Verfahren nach einem der Ansprüche 2 oder 3, **dadurch gekennzeichnet, dass** die Produktfraktion F3 mehr als 95 Gew.-% Polyoxymethylendimethylether mit 3 bis 6 Oxymethylen-Einheiten (OMEₙ₌₃₋₆) enthält.

5. Verfahren nach einem der Ansprüche 1 bis 4 mit den zusätzlichen Schritten:
(v) Einspeisung der Leichtsiederfraktion F1 in einen Apparat zur Wasserabtrennung und Abtrennung von Wasser oder eines wasserreichen Stroms F6, wobei ein wasserarmer Strom F5 erhalten wird;
(vi) gegebenenfalls Rückführung des wasserarmen Stroms F5 in den Reaktor R.

6. Verfahren nach Anspruch 5 mit dem zusätzlichen Schritt:
(vii) Destillation des wasserreichen Stroms F6, wobei ein Methanol und Methylal enthaltender Strom F7 gewonnen und in den Reaktor R zurückgeführt wird, und ein Wasser, Formaldehyd und Polyoxymethylendimethylether mit 2 bis 3 Oxymethylen-Einheiten (OMEₙ₌₂₋₃) enthaltender Strom F8 gewonnen wird.

7. Verfahren nach einem der Ansprüche 2 bis 4, **dadurch gekennzeichnet, dass** die Kolonnen K1 und K2 als eine einzige Reaktivdestillationstrennwandkolonne ausgebildet sind, wobei die Leichtsiederfraktion F1 als Kopfabzugsstrom, die Produktfraktion F3 als Seitenabzugsstrom und die Schwersiederfraktion F4 als Sumpfabzugsstrom der Trennwandkolonne erhalten werden.

8. Verfahren nach einem der Ansprüche 1 bis 7, **dadurch gekennzeichnet, dass** Formaldehyd als wässrige Formaldehyd-Lösung mit einem Formaldehyd-Gehalt von 20 bis 95 Gew.-% in den Reaktor R eingespeist wird.

9. Verfahren nach einem der Ansprüche 1 bis 8, **dadurch gekennzeichnet, dass** Formaldehyd als Paraformaldehyd in Schmelze oder Suspension in den Reaktor R eingespeist wird.

10. Verfahren nach einem der Ansprüche 1 bis 9, **dadurch gekennzeichnet, dass** die Umsetzung in Schritt (i) in Gegenwart eines festen Katalysators, ausgewählt aus der Gruppe bestehend aus lonentauscherharzen, Zeolithen, Aluminosilikaten, Aluminiumdioxid und Titandioxid, durchgeführt wird.

11. Verfahren nach einem der Ansprüche 1 bis 10, **dadurch gekennzeichnet, dass** der pH-Wert in der Reaktivdestillationskolonne K1 von 4 bis 14 beträgt.

12. Verfahren nach einem der Ansprüche 1 bis 11, **dadurch gekennzeichnet, dass** der in Schritt (v) eingesetzte Apparat zur Wasserabtrennung eine Pervaporations-, Dampfpermeations- oder Adsorptionsanlage ist.

13. Verfahren nach einem der Ansprüche 1 bis 12, **dadurch gekennzeichnet, dass** die Reaktivdestillationskolonne im Betrieb folgende Merkmale aufweist:
• der Druck in der Reaktivdestillationskolonne K1 beträgt von 0,2 bar bis 5 bar;
• die Kopftemperatur in der Reaktivdestillationskolonne K1 beträgt von 25°C bis 125°C;
• die Sumpftemperatur in der Reaktivdestillationskolonne K1 beträgt von 110°C bis 255 C;
• die Reaktivdestillationskolonne K1 weist 10 bis 40 theoretische Trennstufen, bevorzugt 15 bis 30 theoretische Trennstufen, besonders bevorzugt 20 bis 25 theoretische Trennstufen auf;
• unterhalb des Zulaufs weist die Reaktivdestillationskolonne K1 3 bis 20 theoretische Trennstufen auf;
• das Rücklaufverhältnis in der Reaktivdestillationskolonne K1 beträgt mindestens 0,20 g/g;
• das Verhältnis aus Volumen der Flüssigkeit im Bereich zwischen Zulauf und Sumpfabzug einschließlich der gegebenenfalls vorhandenen Seitenreaktoren und des Sumpfes zum Volumenstrom des Sumpfabzugs in der Reaktivdestillationskolonne K1 beträgt mindestens 10 min;
• der pH-Wert in der Reaktivdestillationskolonne K1 im Bereich zwischen Zulauf und Sumpfabzug einschließlich der gegebenenfalls vorhandenen Seitenreaktoren und des Sumpfes ist größer als 4.

14. Verfahren nach Anspruch 13, **dadurch gekennzeichnet, dass**
• der Druck in der Reaktivdestillationskolonne K1 von 0,5 bis 4 bar beträgt;
• die Kopftemperatur in der Reaktivdestillationskolonne K1 von 45 bis 115°C beträgt;
• die Sumpftemperatur in der Reaktivdestillationskolonne K1 von 140 bis 240°C beträgt;
• die Reaktionsdestillationskolonne K1 5 bis 30 theoretische Trennstufen aufweist;
• unterhalb des Zulaufs die Reaktivdestillationskolonne K1 4 bis 15 theoretische Trennstufen aufweist;
• der pH-Wert in der Reaktivdestillationskolonne K1 im Bereich zwischen Zulauf und Sumpfabzug größer als 5 ist.

15. Verfahren nach Anspruch 14, **dadurch gekennzeichnet, dass**
• der Druck in der Reaktivdestillationskolonne K1 von 1 bis 3 bar beträgt;
• die Kopftemperatur in der Reaktivdestillationskolonne K1 von 65 bis 105°C beträgt;
• die Sumpftemperatur in der Reaktivdestillationskolonne K1 von 170 bis 225°C beträgt;
• die Reaktionsdestillationskolonne K1 20 bis 25 theoretische Trennstufen aufweist;
• unterhalb des Zulaufs die Reaktivdestillationskolonne K1 5 bis 10 theoretische Trennstufen aufweist;
• der pH-Wert in der Reaktivdestillationskolonne K1 im Bereich zwischen Zulauf und Sumpfabzug zwischen 7 und 14 ist.

## Claims

1. Process for preparing polyoxymethylene dimethyl ethers having ≥ 3 oxymethylene units (OME_{n≥3}), comprising the steps:
(i) introduction of formaldehyde, methanol and water into a reactor R and reaction to give a reaction mixture containing formaldehyde, water, methylene glycol, polyoxymethylene glycols, methanol, hemiformals, methylal (OMEₙ₌₁) and polyoxymethylene dimethyl ethers (OME_{n>1});
(ii) introduction of the reaction mixture into a reactive distillation column K1 and separation into a low boiler fraction F1 containing formaldehyde, water, methylene glycol, polyoxymethylene glycols, methanol, hemiformals, methylal (OMEₙ₌₁) and polyoxymethylene dimethyl ethers having from 2 to 3 oxymethylene units (OMEₙ₌₂₋₃) and a high boiler fraction F2 containing essentially polyoxymethylene dimethyl ethers having more than two oxymethylene units (OME_{n≥3}), wherein the high boiler fraction F2 contains at least 90% by weight of polyoxymethylene dimethyl ethers having more than two oxymethylene units (OME_{n≥3}).

2. Process according to Claim 1 for preparing polyoxymethylene dimethyl ethers having 3 to 6 oxymethylene units (OMEₙ₌₃₋₆), comprising the steps:
(i) introduction of formaldehyde, methanol and water into a reactor R and reaction to give a reaction mixture containing formaldehyde, water, methylene glycol, polyoxymethylene glycols, methanol, hemiformals, methylal (OMEₙ₌₁) and polyoxymethylene dimethyl ethers (OME_{n>1});
(ii) introduction of the reaction mixture into a reactive distillation column K1 and separation into a low boiler fraction F1 containing formaldehyde, water, methylene glycol, polyoxymethylene glycols, methanol, hemiformals, methylal (OMEₙ₌₁) and polyoxymethylene dimethyl ethers having from 2 to 3 oxymethylene units (OMEₙ₌₂₋₃) and a high boiler fraction F2 containing essentially polyoxymethylene dimethyl ethers having more than two oxymethylene units (OME_{n≥3});
(iii) introduction of the high boiler fraction F2 into a distillation column K2 and separation into a product fraction F3 containing polyoxymethylene dimethyl ethers having from 3 to 6 oxymethylene units (OMEₙ₌₃₋₆) and a high boiler fraction F4 containing polyoxymethylene dimethyl ethers having more than 6 oxymethylene units (OME_{n>6});
(iv) optionally recirculation of the high boiler fraction F4 into the reactor R.

3. Process according to Claim 2, **characterized in that** the high boiler fraction F2 contains at least 95% by weight of polyoxymethylene dimethyl ethers having more than two oxymethylene units (OME_{n≥3}).

4. Process according to Claim 2 or 3, **characterized in that** the product fraction F3 contains more than 95% by weight of polyoxymethylene dimethyl ethers having from 3 to 6 oxymethylene units (OMEₙ₌₃₋₆).

5. Process according to any of Claims 1 to 4, comprising the additional steps:
(v) introduction of the low boiler fraction F1 into an apparatus for water separation and removal of water or a water-rich stream F6, giving a low-water stream F5;
(vi) optionally recirculation of the low-water stream F5 into the reactor.

6. Process according to Claim 5 comprising the additional step:
(vii) distillation of the water-rich stream F6 to give a stream F7 which contains methanol and methylal and is recirculated into the reactor R and a stream F8 containing water, formaldehyde and polyoxymethylene dimethyl ethers having from 2 to 3 oxymethylene units (OMEₙ₌₂₋₃).

7. Process according to any of Claims 2 to 4, **characterized in that** the columns K1 and K2 are configured as a single reactive distillation dividing wall column, with the low boiler fraction F1 being obtained as overhead offtake stream, the product fraction F3 being obtained as side offtake stream and the high boiler fraction F4 being obtained as bottom offtake stream from the dividing wall column.

8. Process according to any of Claims 1 to 7, **characterized in that** formaldehyde is introduced as aqueous formaldehyde solution having a formaldehyde content of from 20 to 95% by weight into the reactor R.

9. Process according to any of Claims 1 to 8, **characterized in that** formaldehyde is introduced as paraformaldehyde as melt or suspension into the reactor R.

10. Process according to any of Claims 1 to 9, **characterized in that** the reaction in step (i) is carried out in the presence of a solid catalyst selected from the group consisting of ion-exchange resins, zeolites, aluminosilicates, aluminium dioxide and titanium dioxide.

11. Process according to any of Claims 1 to 10, **characterized in that** the pH in the reactive distillation column K1 is from 4 to 14.

12. Process according to any of Claims 1 to 11, **characterized in that** the apparatus used for water separation in step (v) is a pervaporation, vapour permeation or adsorption plant.

13. Process according to any of Claims 1 to 12, **characterized in that** the reactive distillation column has, in operation, the following features:
• the pressure in the reactive distillation column K1 is from 0.2 bar to 5 bar;
• the temperature at the top of the reactive distillation column K1 is from 25°C to 125°C;
• the temperature at the bottom of the reactive distillation column K1 is from 110°C to 255°C;
• the reactive distillation column K1 has from 10 to 40 theoretical plates, preferably from 15 to 30 theoretical plates, particularly preferably from 20 to 25 theoretical plates;
• below the feedpoint, the reactive distillation column K1 has from 3 to 20 theoretical plates;
• the reflux ratio in the reactive distillation column K1 is at least 0.20 g/g;
• the ratio of volume of the liquid in the region between feedpoint and bottom offtake including any side reactors present and the bottom to the volume flow of the bottom offtake stream in the reactive distillation column K1 is at least 10 min;
• the pH in the reactive distillation column K1 in the region between feedpoint and bottom offtake including any side reactors present and the bottom is greater than 4.

14. Process according to Claim 13, **characterized in that**
• the pressure in the reactive distillation column K1 is from 0.5 to 4 bar;
• the temperature at the top of the reactive distillation column K1 is from 45 to 115°C;
• the temperature at the bottom of the reactive distillation column K1 is from 140 to 240°C;
• the reactive distillation column K1 has from 5 to 30 theoretical plates;
• below the feedpoint, the reactive distillation column K1 has from 4 to 15 theoretical plates;
• the pH in the reactive distillation column K1 in the region between feedpoint and bottom offtake is greater than 5.

15. Process according to Claim 14, **characterized in that**
• the pressure in the reactive distillation column K1 is from 1 to 3 bar;
• the temperature at the top of the reactive distillation column K1 is from 65 to 105°C;
• the temperature at the bottom of the reactive distillation column K1 is from 170 to 225°C;
• the reactive distillation column K1 has from 20 to 25 theoretical plates;
• below the feedpoint, the reactive distillation column K1 has from 5 to 10 theoretical plates;
• the pH in the reactive distillation column K1 in the region between feedpoint and bottom offtake is in the range from 7 to 14.

## Revendications

1. Procédé pour la préparation de polyoxyméthylènediméthyléthers dotés de ≥ 3 motifs oxyméthylène (OME_{n ≥ 3}) comportant les étapes de :
(i) introduction de formaldéhyde, de méthanol et d'eau dans un réacteur R et transformation en un mélange réactionnel contenant du formaldéhyde, de l'eau, du méthylèneglycol, des polyoxyméthylèneglycols, du méthanol, des hémiformals, du méthylal (OME_{n = 1}) et des polyoxyméthylènediméthyléthers (OME_{n > 1}) ;
(ii) introduction du mélange réactionnel dans une colonne de distillation réactive K1 et séparation en une fraction de composés volatils F1 contenant du formaldéhyde, de l'eau, du méthylèneglycol, des polyoxyméthylèneglycols, du méthanol, des hémiformals, du méthylal (OME_{n = 1}) et des polyoxyméthylènediméthyléthers comportant 2 à 3 motifs oxyméthylène (OME_{n = 2 à 3}) et en une fraction de composés non volatils F2 contenant des polyoxyméthylènediméthyléthers comportant plus de deux motifs oxyméthylène (OME_{n ≥ 3}), la fraction de composés non volatils F2 contenant au moins 90 % en poids de polyoxyméthylènediméthyléthers comportant plus de deux motifs oxyméthylène (OME_{n ≥ 3}).

2. Procédé selon la revendication 1 pour la préparation de polyoxyméthylènediméthyléthers comportant 3 à 6 motifs oxyméthylène (OME_{n = 3 à 6}) comportant les étapes de :
(i) introduction de formaldéhyde, de méthanol et d'eau dans un réacteur R et transformation en un mélange réactionnel contenant du formaldéhyde, de l'eau, du méthylèneglycol, des polyoxyméthylèneglycols, du méthanol, des hémiformals, du méthylal (OME_{n = 1}) et des polyoxyméthylènediméthyléthers (OME_{n > 1}) ;
(ii) introduction du mélange réactionnel dans une colonne de distillation réactive K1 et séparation en une fraction de composés volatils F1 contenant du formaldéhyde, de l'eau, du méthylèneglycol, des polyoxyméthylèneglycols, du méthanol, des hémiformals, du méthylal (OME_{n = 1}) et des polyoxyméthylènediméthyléthers comportant 2 à 3 motifs oxyméthylène (OME_{n = 2 à 3}) et en une fraction de composés non volatils F2 contenant des polyoxyméthylènediméthyléthers comportant plus de deux motifs oxyméthylène (OME_{n ≥ 3}), la fraction de composés non volatils F2 contenant au moins 90 % en poids de polyoxyméthylènediméthyléthers comportant plus de deux motifs oxyméthylène (OME_{n ≥ 3});
(iii) introduction de la fraction de composés non volatils F2 dans une colonne de distillation K2 et séparation en une fraction de produit F3 contenant des polyoxyméthylènediméthyléthers comportant 3 à 6 motifs oxyméthylène (OME_{n = 3 à 6}) et en une fraction de composés non volatils F4 contenant des polyoxyméthylènediméthyléthers comportant plus de 6 motifs oxyméthylène (OME_{n > 6}) ;
(iv) éventuellement recyclage de la fraction de composés non volatils F4 dans le réacteur R.

3. Procédé selon la revendication 2, **caractérisé en ce que** la fraction de composés non volatils F2 contient au moins 95 % en poids de polyoxyméthylènediméthyléthers comportant plus de deux motifs oxyméthylène (OME_{n ≥ 3}).

4. Procédé selon l'une quelconque des revendications 2 et 3, **caractérisé en ce que** la fraction de produit F3 contient plus de 95 % en poids de polyoxyméthylènediméthyléthers comportant 3 à 6 motifs oxyméthylène (OME_{n = 3 à 6}).

5. Procédé selon l'une quelconque des revendications 1 à 4 comportant les étapes supplémentaires de :
(v) introduction de la fraction de composés volatils F1 dans un appareil pour la séparation d'eau et séparation de l'eau ou d'un flux riche en eau F6, un flux pauvre en eau F5 étant obtenu ;
(vi) éventuellement recyclage du flux pauvre en eau F5 dans le réacteur R.

6. Procédé selon la revendication 5 comportant l'étape supplémentaire de :
(vii) distillation du flux riche en eau F6, un flux F7 contenant du méthanol et du méthylal étant obtenu et étant recyclé dans le réacteur R, et un flux F8 contenant de l'eau, du formaldéhyde et des polyoxyméthylènediméthyléthers comportant 2 à 3 motifs oxyméthylène (OME_{n = 2 à 3}) étant obtenu.

7. Procédé selon l'une quelconque des revendications 2 à 4, **caractérisé en ce que** les colonnes K1 et K2 sont formées en tant qu'une seule colonne de distillation réactive à paroi de séparation, la fraction de composés volatils F1 étant obtenue en tant que flux d'échappement de tête, la fraction de produit F3 étant obtenue en tant que flux d'échappement latéral et la fraction de composés non volatils F4 étant obtenue en tant que flux d'échappement de fond de la colonne à paroi de séparation.

8. Procédé selon l'une quelconque des revendications 1 à 7, **caractérisé en ce que** le formaldéhyde est introduit dans le réacteur R en tant que solution aqueuse de formaldéhyde dotée d'une teneur en formaldéhyde de 20 à 95 % en poids.

9. Procédé selon l'une quelconque des revendications 1 à 8, **caractérisé en ce que** le formaldéhyde est introduit dans le réacteur R en tant que paraformaldéhyde sous forme de masse fondue ou en suspension.

10. Procédé selon l'une quelconque des revendications 1 à 9, **caractérisé en ce que** la transformation dans l'étape (i) est mise en œuvre en présence d'un catalyseur solide, choisi dans le groupe constitué par des résines échangeuses d'ions, des zéolithes, des aluminosilicates, du dioxyde d'aluminium et du dioxyde de titane.

11. Procédé selon l'une quelconque des revendications 1 à 10, **caractérisé en ce que** la valeur du pH dans la colonne de distillation réactive K1 est de 4 à 14.

12. Procédé selon l'une quelconque des revendications 1 à 11, **caractérisé en ce que** l'appareil utilisé dans l'étape (v) pour la séparation d'eau est une installation de pervaporation, de perméation de vapeur ou d'adsorption.

13. Procédé selon l'une quelconque des revendications 1 à 12, **caractérisé en ce que** la colonne de distillation réactive en fonctionnement présente les caractéristiques suivantes :
• la pression dans la colonne de distillation réactive K1 est de 0,2 à 5 bars ;
• la température de tête dans la colonne de distillation réactive K1 est de 25 °C à 125 °C;
• la température de fond dans la colonne de distillation réactive K1 est de 110 °C à 255 °C;
• la colonne de distillation réactive K1 présente 10 à 40 plateaux théoriques, préférablement 15 à 30 plateaux théoriques, particulièrement préférablement 20 à 25 plateaux théoriques ;
• la colonne de distillation réactive K1 présente en dessous de l'alimentation 3 à 20 plateaux théoriques ;
• le rapport de reflux dans la colonne de distillation réactive K1 est d'au moins 0,20 g/g ;
• le rapport du volume du liquide dans la zone entre l'alimentation et la sortie de fond y compris les réacteurs latéraux éventuellement présents et le fond sur le flux de volume de la sortie de fond dans la colonne de distillation réactive K1 est d'au moins 10 min ;
• la valeur du pH dans la colonne de distillation réactive K1 dans la zone entre l'alimentation et la sortie de fond y compris les réacteurs latéraux éventuellement présents et le fond est supérieure à 4.

14. Procédé selon la revendication 13, **caractérisé en ce que**
• la pression dans la colonne de distillation réactive K1 est de 0,5 à 4 bars ;
• la température de tête dans la colonne de distillation réactive K1 est de 45 à 115 °C ;
• la température de fond dans la colonne de distillation réactive K1 est de 140 à 240 °C ;
• la colonne de distillation réactive K1 présente 5 à 30 plateaux théoriques ;
• la colonne de distillation réactive K1 présente en dessous de l'alimentation 4 à 15 plateaux théoriques ;
• la valeur du pH dans la colonne de distillation réactive K1 dans la zone entre l'alimentation et la sortie de fond est supérieure à 5.

15. Procédé selon la revendication 14, **caractérisé en ce que**
• la pression dans la colonne de distillation réactive K1 est de 1 à 3 bars ;
• la température de tête dans la colonne de distillation réactive K1 est de 65 à 105 °C ;
• la température de fond dans la colonne de distillation réactive K1 est de 170 à 225 °C ;
• la colonne de distillation réactive K1 présente 20 à 25 plateaux théoriques ;
• la colonne de distillation réactive K1 présente en dessous de l'alimentation 5 à 10 plateaux théoriques ;
• la valeur du pH dans la colonne de distillation réactive K1 dans la zone entre l'alimentation et la sortie de fond est comprise entre 7 et 14.
